# EUROPEAN PATENT SPECIFICATION

(11) **EP 4 536 849 B1**
(45) Date of publication and mention of the grant of the patent: **01.07.2026**
(21) Application number: 23739402.8
(22) Date of filing: 06.06.2023
(51) Int. Cl.: C12Q 1/6806

(54) **COMBINATORIAL INDEXING FOR SINGLE-CELL NUCLEIC ACID SEQUENCING**
KOMBINATORISCHE INDEXIERUNG ZUR EINZELZELL-NUKLEINSÄURESEQUENZIERUNG
INDEXATION COMBINATOIRE POUR LE SÉQUENÇAGE DE L'ACIDE NUCLÉIQUE MONOCELLULAIRE

(30) Priority: 06.06.2022 US 202263349184 P; 27.01.2023 US 202363441744 P; 02.05.2023 US 202363499689 P
(43) Date of publication of application: 16.04.2025
(73) Proprietor: GENENTECH, INC., South San Francisco, CA 94080 (US)
(72) Inventor: WU, Bing, South San Francisco, CA 94080-4990 (US); DARMANIS, Spryidon, South San Francisco, CA 94080-4990 (US); MODRUSAN, Zora, South San Francisco, CA 94080-4990 (US); BENNETT, Hayley Marie, South San Francisco, CA 94080-4990 (US)
(74) Representative: Küng, Peter
(86) International application number: PCT/US2023/024597
(87) International publication number: WO 2023/239733

(56) References cited:
- WO-A1-2020/214642
- WO-A1-2021/044063
- WO-A1-2021/046475
- WO-A1-2021/188838

## Description

### FIELD

The present disclosure relates to methods for combinatorial indexing of nucleic acids of single cells or nuclei. The present disclosure further provides methods for generating and sequencing libraries of such indexed nucleic acids.

### BACKGROUND

Sequencing of nucleic acids of single cells have emerged as a powerful tool for analyzing the genetic heterogeneity of a group of cells. For example, sequencing nucleic acids of single cells can be used to analyze the progression of cancer, to map cellular heterogeneity in diseased and healthy tissues, to understand the development of immunity or autoimmune disorders and to gain a better understanding of natural biological processes.

Single cell sequencing typically includes the addition of unique barcodes to the nucleic acids of single cells to allow profiling of many single cells in parallel. There are several techniques for adding barcodes to nucleic acids of single cells and typically include the use of multi-well plates or microfluidic devices. However, such techniques are limited by the capacity of the plate or microfluidic device to avoid the loading of more than one cell in a well of the multi-well plate or droplet generated by the microfluidic device and prevent the labeling of nucleic acids of multiple cells with the same barcode. Therefore, there is a need in the art for improved high throughput methods for indexing nucleic acids of single cells.

WO 2021/046475 discloses a hybrid workflow that combines split-pool combinatorial barcoding with partition-based microfluidics to analyse intracellular analytes. First, individual cells (or cell beads) are molecularly indexed on their outer surface using a split-pool method to create a unique composite barcode on the cell surface. These surface-barcoded cells are then encapsulated into partitions, such as droplets or wells, where they are lysed to release both the surface barcodes and the intracellular analytes (such as RNA or DNA). Finally, the released barcodes tag the intracellular analytes within the partition, which allows the analytes to be traced back to their originating cell even if multiple cells were loaded into a single partition, thereby overcoming the throughput limitations of Pois-son statistics.

### SUMMARY

The present disclosure provides for methods for combinatorial indexing of nucleic acids of single cells and methods for generating and sequencing libraries of such indexed nucleic acids. In one aspect, the present invention provides a method for combinatorial indexing of nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence; and
(c) incorporating the second index sequence into the nucleic acids in each compartment to generate dual-indexed nucleic acids.
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;
(iv) incorporating the first index sequence into nucleic acids of the cells or nuclei to generate indexed nucleic acids; and
(v) combining the cells or nuclei that associate with or comprise the indexed nucleic acids from a plurality of the emulsion droplets to generate the pooled cells or nuclei associated with or comprising nucleic acids comprising a first index sequence.

In another aspect, the present invention provides a method for generating a sequencing library comprising nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence;
(c) incorporating the second index sequence into the nucleic acids in each compartment to generate dual-indexed nucleic acids; and
(d) combining all or a subset of the dual-indexed nucleic acids to generate a sequencing library from the plurality of cells;
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;
(iv) incorporating the first index sequence into nucleic acids of the cells or nuclei to generate indexed nucleic acids; and
(iv) combining the cells or nuclei that associate with or comprise the indexed nucleic acids from a plurality of the emulsion droplets to generate the pooled cells or nuclei associated with or comprising nucleic acids comprising a first index sequence.

In another aspect, the present invention provides a method for sequencing a library comprising nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence;
(c) incorporating the second index sequence into the nucleic acids in each compartment to generate dual-indexed nucleic acids;
(d) combining all or a subset of the dual-indexed nucleic acids to generate a sequencing library from the plurality of cells; and
(e) sequencing all or a subset of the dual-index nucleic acids;
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;;
(iv) incorporating the first index sequence into nucleic acids of the cells or nuclei to generate indexed nucleic acids; and
(v) combining the cells or nuclei that associate with or comprise the indexed nucleic acids from a plurality of the emulsion droplets to generate the pooled cells or nuclei associated with or comprising nucleic acids comprising a first index sequence.

In certain embodiments of the present invention, the cells or nuclei are treated with a T5 transposase prior to or during step (i).

In certain embodiments of the present invention, the plurality of cells are permeabilized or lysed prior to step (i).

In certain embodiments of the present invention,t he plurality of cells or nuclei are treated with a multiplexing reagent prior to or during step (i).

In certain embodiments of the present invention, the first index sequence of each particle or a subset of particles is unique relative to other particles.

In certain embodiments of the present invention, the second index sequence in one compartment or a subset of compartments is unique relative to other second index sequences.

In certain embodiments of the present invention, the plurality of compartments is a multi-well plate.

In certain embodiments of the present invention, the emulsion droplets are generated in an emulsion droplet generating device or generated using an emulsion droplet generating technique.

In certain embodiments of the present invention, the method further comprising incorporating a third index sequence into the dual-indexed nucleic acids to generate triple-indexed nucleic acids.

In certain embodiments of the present invention, incorporating the first index sequence into the nucleic acids in emulsion droplets is performed by an amplification process, a reverse-transcription process or a ligation process.

In certain embodiments of the present invention, incorporating the second index sequence into the indexed nucleic acids is performed by an amplification process or a ligation process.

In certain embodiments of the present invention, the amplification process is polymerase chain reaction (PCR) or an isothermal amplification process.

In certain embodiments of the present invention,the plurality of cells comprises pluripotent stem cells, embryonic stem cells, somatic cells, immune cells, cancer cells or a combination thereof.

### BRIEF DESCRIPTION OF THE FIGURES

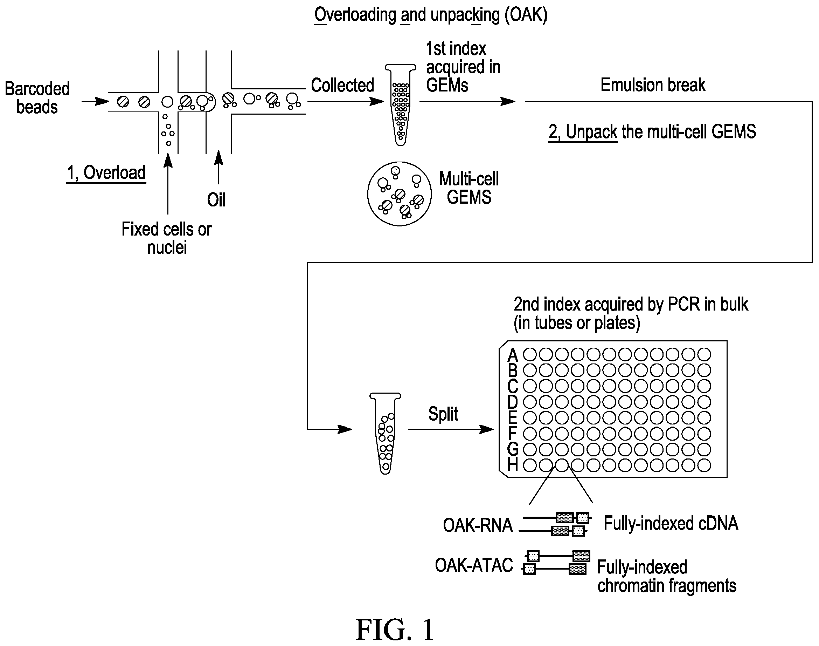

**FIG. 1** is a schematic showing an exemplary embodiment of the overloading and unpacking ("OAK") method for combinatorial indexing of nucleic acids of single cells or nuclei. The OAK method comprises overloading cells or nuclei in an emulsion droplet generating device along with gel beads to generate multi-cell gel bead-in-emulsion ("GEMS") (left) to incorporate a first index sequence in the nucleic acids of the cells or nuclei. The multi-cell GEMS are subsequently unpacked to generate pooled cells or nuclei with indexed nucleic acids (middle). The pooled indexed cells or nuclei are then distributed into different compartments to acquire a second index sequence (right) to generate dual-indexed nucleic acids.
**FIG. 2** provides an image showing multi-cell GEMs generated from the loading of a sample that included 280,000 Jurkat cells into an emulsion droplet generating device.
**FIG. 3** provides an image showing intact Jurkat cells that were recovered after unpacking of the multi-cell GEMs.
**FIG. 4** provides a schematic of an exemplary method that includes a single-cell CRISPR screen and an OAK method of the present disclosure.
**FIG. 5** provides a schematic of an exemplary method that includes a lineage tracing technique and an OAK method of the present disclosure.
**FIG. 6** provides a schematic showing the loading of different types of gel beads (*e.g.,* 3' gel beads, 5' gel beads or multiome gel beads) into an emulsion droplet generating device along with cells to generate multi-cell GEMS for performing an exemplary OAK-multiome method or an exemplary OAK-scRNAseq method of the present disclosure.
**FIG. 7A** provides an image showing multi-cell GEMs generated from the loading of a sample that included 150,000 cells into an emulsion droplet generating device.
**FIG. 7B** provides an image showing multi-cell GEMs generated from the loading of a sample that included 450,000 cells into an emulsion droplet generating device.
**FIG. 8** provides the performance metrics for an exemplary OAK-multiome method of the present disclosure.
**FIG. 9** provides a schematic of an exemplary method of performing a cell hashing technique in combination with the OAK method of the present disclosure. Created with BioRender. com.
**FIG. 10** provides a graph showing the abundance of cells assigned the 9 different hashtags in sequencing data obtained using the OAK method compared to a control method on the same cell sample.
**FIG. 11** provides ridge plots showing hashtag expression level for cells deplexed for each hashtag identity.
**FIG. 12** provides the quality of the transcriptome sequencing data from the experiment set forth in **FIG. 9****.**
**FIGS. 13A-13B** provides UMAPs of scRNA-Seq with hashtag assignment displayed **(****FIG. 13A****)** showing that the hashtags are evenly distributed amongst different cell types **(****FIG. 13B****).**
**FIG. 13C** provides a graph showing cell type frequency obtained using the OAK method compared to a control method on the same cell sample.
**FIG. 14** provides a schematic of an exemplary method of performing a cell hashing technique in combination with the OAK method of the present disclosure for two cell lines, *i.e.,* the Jurkat and K562 cell lines. Created with BioRender.com.
**FIG. 15** provides the quality of the transcriptome sequencing data obtained from the experiment set forth in **FIG. 14****.**
**FIG. 16** provides a UMAP showing the clustering of the scRNA-Seq data from the Jurkat and K562 cell lines.
**FIG. 17** provides plots showing the signal derived from the antibody hashtags of the Jurkat and K562 cell lines.
**FIG. 18** shows that the frequency of the CDR3 clonotype recovered from a homogeneous population of Jurkat cells.

### DETAILED DESCRIPTION

The present disclosure relates to improved methods for combinatorial indexing of nucleic acids from single cells. In certain embodiments, methods of the present disclosure comprise the incorporation of at least two index sequences into the nucleic acids of a single cell or nuclei. By using at least two index sequences, the combinatorial indexing method of the present disclosure allows the identification of nucleic acids from a single cell or nuclei by the presence of a unique combination of the two index sequences. In certain embodiments, methods of the present disclosure can be used to generate libraries of the indexed nucleic acids, *e.g*., dual-indexed nucleic acids. In certain embodiments, the present disclosure further provides methods for sequencing the libraries generated from indexed nucleic acids, *e.g*., dual-indexed nucleic acids.

The present disclosure is based, in part, on the discovery that by overloading emulsion droplets to contain at least two cells or nuclei for incorporating a first index sequence into the nucleic acids of the cells or nuclei increases the throughput of the indexing method by more than 20-fold. For example, by overloading emulsion droplets with more than one cell or nuclei for incorporating the first index sequence, the methods of the present disclosure allow the combinatorial indexing of nucleic acids from more than 200,000 single cells. In addition, by using emulsion droplets as the first step in adding index sequences to the nucleic acids of single cells, the disclosed combinatorial indexing process allows the cells to remain intact for the addition of the second index sequence to the indexed nucleic acids. Furthermore, the use of emulsion droplets as the first step of the indexing process ensures compatibility with different emulsion-based kits, reagents and methods, and allows for subsets of cells to be sequenced independently of each other.

The nucleic acids to be indexed using the methods of the present disclosure can be DNA or RNA. Methods for the present disclosure can be used to index the RNAs of a single cell. For example, but not by way of limitation, methods for the present disclosure can be used to analyze all or part of the transcriptome of a single cell by indexing the mRNAs of the single cell. In certain embodiments, methods of the present disclosure can be used to index genomic DNA of a single cell or nuclei. Methods of the present disclosure can be used to analyze gene regulation in a single cell by indexing the genomic DNA of the single cell. Methods of the present disclosure can be used to analyze chromatin accessibility in a single cell by indexing the open chromatin sites in the genomic DNA of the single cell. Methods of the present disclosure can be used to analyze multiple samples that have been pooled and indexed using sample-specific oligonucleotides prior to pooling or are genetically distinct. Methods of the present disclosure can be used to quantify the presence of proteins following protein detection using oligonucleotide labeled antibodies.

For clarity, but not by way of limitation, the detailed description of the presently disclosed subject matter is divided into the following subsections:
I. Definitions;
II. Cells;
III. Method of Use;

### I. DEFINITIONS

Unless defined otherwise, all technical and scientific terms used herein have the meaning commonly understood by a person skilled in the art to which the subject matter of the present disclosure belongs. The following references provide one of skill with a general definition of many of the terms used in the present disclosure: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, the use of the word "a" or "an" when used in conjunction with the term "comprising" in the claims and/or the specification can mean "one," but it is also consistent with the meaning of "one or more," "at least one," and "one or more than one."

The term "about" or "approximately" means within an acceptable error range for the particular value as determined by one of ordinary skill in the art, which depends in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

The term "amplification process" refers generally to any process where a portion of a nucleic acid is copied or replicated into at least one additional nucleic acid molecule.

The term "antibody" herein is used in the broadest sense and encompasses various antibody structures, including but not limited to monoclonal antibodies, polyclonal antibodies, multispecific antibodies (*e.g*., bispecific antibodies) and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (*e.g*., scFv) and multispecific antibodies formed from antibody fragments.

As used herein, the term "biological sample" refers to a sample of biological material obtained from a subject, including a biological fluid, *e.g.,* blood, plasma, serum, urine, sputum, spinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, tumor cyst fluid, amniotic fluid, bronchoalveolar fluid, biliary fluid and combinations thereof.

The term "combinatorial indexing" refers to the process of incorporating two or more index sequences into a nucleic acid as an identifying sequence.

As used herein, the term "compartment" refers to an area or volume that separates or isolates one or more components from other components. Non-limiting examples of compartments include vials, tubes, wells, droplets, boluses, vessels or areas or volumes separated by physical forces such as fluid flow, magnetism, electrical current or the like.

The terms "comprise(s)," "include(s)," "having," "has," "can," "contain(s)" and variants thereof, as used herein, are intended to be open-ended transitional phrases, terms or words that do not preclude additional acts or structures. The present disclosure also contemplates other embodiments "comprising," "consisting of" and "consisting essentially of," the embodiments or elements presented herein, whether explicitly set forth or not.

The term "coupled" can refer to the connecting or uniting of two or more components by an interaction, bond, link, force or tie in order to keep two or more components together. The term "coupled" encompasses either direct or indirect binding where, for example, a first component is directly bound to a second component, or one or more intermediate molecules are disposed between the first component and the second component. Exemplary bonds comprise covalent bonds, ionic bonds, van der Waals interactions, hydrogen bonds and other bonds identifiable by a skilled person.

The term "deplex," as used herein, refers to the separation of different data obtained during a single experiment. The term "deplex" refers to the separation of scRNA-SEQ data from hashtag sequencing and/or expression data.

The terms "detect" or "detection," as used herein, indicate the determination of the existence and/or presence of a target, *e.g*., a protein target or a nucleic acid target, in a limited portion of space, including but not limited to a sample. The terms "detect" or "detection," as used herein, can comprise determination of chemical and/or biological properties of the target, including but not limited to ability to interact, and in particular bind, other compounds, ability to activate another compound and additional properties identifiable by a skilled person upon reading of the present disclosure. The detection can be quantitative or qualitative. A detection is "quantitative" when it refers, relates to, or involves the measurement of quantity or amount of the target or signal (also referred as quantitation), which includes but is not limited to any analysis designed to determine the amounts or proportions of the target or signal. A detection is "qualitative" when it refers, relates to, or involves identification of a quality or kind of the target or signal in terms of relative abundance to another target or signal, which is not quantified.

As used herein, the term "dual-indexed nucleic acids" refers to nucleic acids with two index sequences.

As used herein, the term "emulsion droplet" refers to droplets formed by at least two immiscible phases, *e.g.,* a continuous phase and a dispersed phase. The emulsion droplets can be formed in an emulsion droplet generating device using the addition of oil as the continuous phase into the emulsion droplet generating device. The emulsion droplets can be formed by a technique that produces emulsion droplets such as, but not limited to, vortexing.

As used herein, the term "hybridization," refers to the process in which two single-stranded polynucleotides bind non-covalently to form a stable double-stranded polynucleotide.

As used herein, the term "index sequence" refers to a nucleotide sequence used to identify a single cell or nuclei with which the index sequence is associated.

As used herein, the term "individual" or "subject" refers to a vertebrate or an invertebrate, such as a human or non-human animal, for example, a mammal. Mammals include, but are not limited to, humans, non-human primates, farm animals, sport animals, rodents and pets. Non-limiting examples of non-human animal subjects include rodents such as mice, rats, hamsters, guinea pigs, rabbits, dogs, cats, sheep, pigs, goats, cattle, horses, apes and monkeys. The individual or subject can be a human.

As used herein, the term *"in vitro"* refers to an artificial environment and to processes or reactions that occur within an artificial environment. In vitro environments exemplified, but are not limited to, test tubes and cell cultures.

As used herein, the term *"in vivo"* refers to the natural environment (*e.g.,* an animal or a cell) and to processes or reactions that occur within a natural environment, such as embryonic development, cell differentiation, neural tube formation, etc.

The term "isolated nuclei," as used herein, refers to nuclei that have been separated from a component of its natural environment.

An "isolated nucleic acid" refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "ligation," as used herein, refers to the formation of a covalent bond or linkage between two or more molecules, e.g., between the termini of two or more nucleic acid molecules.

As used herein, the term "ligation process" refers generally to a process for covalently linking two or more molecules together by an enzyme. For example, two or more nucleic acid molecules can be covalently linked together by a ligation process using a ligase.

As used herein, a "marker" refers to an agent that allows for direct or indirect detection. Markers include, but are not limited to, fluorescent labels, chromogenic labels, electron dense labels, chemiluminescent labels and radioactive labels. Non-limiting examples of markers include green fluorescent protein ("GFP"), mCherry, dtTomato, or other fluorescent proteins known in the art (*e*.*g*., Shaner et al., A Guide to Choosing Fluorescent Proteins, Nature Methods 2(12):905-909 (2005), 32P,14C,125I, 3H and 131I, fluorogens (such as Rare Earth Chelate or lucifer yellow and its derivatives), Rhodamine (rhodamine) and its derivatives, dansyl, umbelliferone, luciferase (such as firefly luciferase and bacterial fluorescence plain enzyme) (U.S. Patent number 4,737,456), fluorescein, 2,3-dihydros phthalazine diketone, as well as enzymes producing detectable signals, *e.g*., horseradish peroxidase (HRP), alkaline phosphorus sour enzyme, beta galactosidase, glucoamylase, lysozyme, carbohydrate oxidase (such as glucose oxidase, galactose oxidase and glucose-6-phosphate dehydrogenase (G6PD)), and heterocyclic oxidases (such as uricase and xanthine oxidase).

As used herein, the term "multi-indexed" refers to nucleic acids with at least two index sequences.

The term "nucleic acid" or "polynucleotide" includes any compound and/or substance that comprises a polymer of nucleotides. Each nucleotide is composed of a base, specifically a purine- or pyrimidine base (*i.e.,* cytosine (C), guanine (G), adenine (A), thymine (T) or uracil (U)), a sugar (*i.e.,* deoxyribose or ribose), and a phosphate group. Often, the nucleic acid molecule is described by the sequence of bases, whereby said bases represent the primary structure (linear structure) of a nucleic acid molecule. The sequence of bases is typically represented from 5' to 3'. The term nucleic acid encompasses deoxyribonucleic acid (DNA) including, e.g., complementary DNA (cDNA) and genomic DNA, ribonucleic acid (RNA), e.g., messenger RNA (mRNA), synthetic forms of DNA or RNA, and mixed polymers comprising two or more of these molecules. The nucleic acid molecule can be linear or circular. In addition, the term nucleic acid includes both, sense and antisense strands, as well as single stranded and double stranded forms. Moreover, the herein described nucleic acid can contain naturally occurring or non-naturally occurring nucleotides. Examples of non-naturally occurring nucleotides include modified nucleotide bases with derivatized sugars or phosphate backbone linkages or chemically modified residues.

As used herein, the term "partitioning" refers generally to the separation of one or more components from other components in an area or volume.

The term "plurality" refers to a number larger than one. The term "plurality of cells" refers to a number of cells larger than one. For example, but not by way of limitation, a plurality of cells includes at least two cells. A plurality of cells includes about 10,000 or more cells, about 20,000 or more cells, about 30,000 or more cells, about 40,000 or more cells, about 50,000 or more cells, about 100,000 or more cells, about 150,000 or more cells, about 200,000 or more cells, about 300,000 or more cells, about 400,000 or more cells or 500,000 or more cells. A plurality of cells includes at least about 100,000 cells. The term "plurality of particles" refers to a number of particles larger than one.

The term "reverse-transcription process" refers to a process of generating a complementary strand of DNA using an enzyme called a reverse transcriptase.

The term "specifically binds," as used herein, refers to the preferential binding to a target molecule, *e.g.,* a protein or nucleic acid, relative to other molecules, *e.g.,* proteins or nucleic acids, in a sample.

As used herein, the term "subset" refers to a small portion of a larger quantity of material.

As used herein, the term "triple-indexed" refers to nucleic acids with three index sequences.

### II. CELLS

The present disclosure provides methods for indexing nucleic acids, *e.g*., dual-indexing nucleic acids, of single cells or nuclei obtained from single cells. The cells for use in the presently disclosed methods can include any type of cell. Nuclei for use in the presently disclosed methods can be obtained, *e.g*., isolated, from any type of cell.

The cells can be obtained from a subject. The subject can be a human, non-human primate, *e.g*., an ape or a monkey, a farm animal, a mouse, a rat, a hamster, a guinea pig, a rabbit, a dog, cat, a sheep, a pig, a goat, a cow or a horse.

The cells can be obtained from a biological fluid. Non-limiting examples of biological fluids include whole blood, plasma, serum, sweat, urine, sputum, spinal fluid, pleural fluid, mucus, nipple aspirates, lymph fluid, fluid of the respiratory, intestinal and genitourinary tracts, interstitial fluid, tear fluid, saliva, breast milk, fluid from the lymphatic system, semen, vaginal secretions, cerebrospinal fluid, intra-organ system fluid, ascitic fluid, tumor cyst fluid, amniotic fluid, bronchoalveolar fluid, biliary fluid and combinations thereof.

The cells can be obtained from a tissue, *e.g.,* a tissue sample. Non-limiting examples of tissues include eye (*e.g*., retina), muscle, skin, tendon, vein, artery, blood, heart, spleen, lymph node, bone, bone marrow, lung, bronchi, trachea, gut, small intestine, large intestine, colon, rectum, salivary gland, tongue, gallbladder, appendix, liver, pancreas, brain, stomach, skin, kidney, ureter, bladder, urethra, gonad, testicle, ovary, uterus, fallopian tube, thymus, pituitary, thyroid, adrenal or parathyroid tissue.

The cells for use in the present disclosure can be fetal cells, e.g., obtained from fetal tissue and/or amniotic fluid. The methods of the present disclosure can be used to analyze fetal health and/or identify an abnormality in individual fetal cells.

The cells can be obtained from *in vitro* cell cultures. For example, but not by way of limitation, the cells can include cell lines.

The cells can include primary cells, blood cells, somatic cells, cancer cells, cells derived from organoids or xenografts or stem cells, *e.g*., pluripotent stem cells (iPSCs) or embryonic stem cellsThe cells for use in the present disclosure can be derived from stem cells, *e.g*., stem cells that have undergone natural differentiation or artificially induced reprogramming or transdifferentiation.

The cells can be obtained from preserved samples, *e.g*., fixed samples, from frozen samples, *e.g.,* frozen tissue samples, or from fresh samples, *e.g.,* fresh tissue samples.

The cells for use in the present disclosure can be treated with an agent. For example, but not by way of limitation, the agent can be a therapeutic agent. The cells for use of the present disclosure can be contacted with the agent, *e.g*., therapeutic agent, prior to being subjected to a combinatorial indexing method described herein. Methods of the present disclosure can be used in drug screening, *e.g*., to determine the genomic and/or transcriptional changes associated with a test therapeutic agent, *e.g*., a newly identified therapeutic agent. Methods of the present disclosure can be used in determining the genomic and/or transcriptional changes associated with resistance to a therapeutic agent. Non-limiting examples of such therapeutics include polypeptide therapeutics, *e.g*., antibody-based therapeutics, oligonucleotides, cell-based therapeutics, gene editing systems, and small molecule therapeutics. The therapeutic agent can be cell cycle regulators, kinase regulators (*e.g*., kinase inhibitors or activators), receptor regulators (*e.g*., receptor inhibitors or activators), chemotherapeutics and/or antibodies (*e.g*., agonist or antagonist antibodies). A method of the present disclosure can include performing a single-cell therapeutic agent screen followed by a combinatorial indexing method described herein.

The cells can be genetically modified to express and/or secrete an agent, e.g., a therapeutic agent. Non-limiting examples of therapeutics are described herein. For example, but not by way of limitation, the cells to be used in the methods of the present disclosure express a polypeptide therapeutic, *e.g*., an antibody or an antibody fragment.

The cells can be immune cells. Non-limiting examples of immune cells include neutrophils, eosinophils, basophils, mast cells, monocytes, macrophages, dendritic cells, natural killer cells (NK cells) and lymphocytes, *e.g.,* B cells and T cells (*e.g.,* cytotoxic T cells, natural killer T cells, regulatory T cells and helper T cells). The cell can be a T cell. The cells can be modified immune cells, *e.g.,* modified T cells, that have been genetically engineered to express a chimeric antigen receptor (CAR), *e.g.,* CAR T cells and CAR NK cells.

The cells can be obtained from a malignancy of a tissue or a tumor. Non-limiting examples of such malignancies include carcinomas, adenocarcinomas, sarcomas and fibroadenomas. The cells are obtained from a cancer such as bladder cancer, bone cancer, brain cancer, breast cancer, cervical cancer, colorectal cancer, head and neck cancer, kidney cancer, leukemia, lung cancer, lymphoma, melanoma, pancreatic cancer, parathyroid cancer, prostate cancer, stomach cancer, testicular cancer, thyroid cancer and uterine cancer. The methods of the present disclosure can be used to identify mutations and/or gene alterations present in single cancer cells.

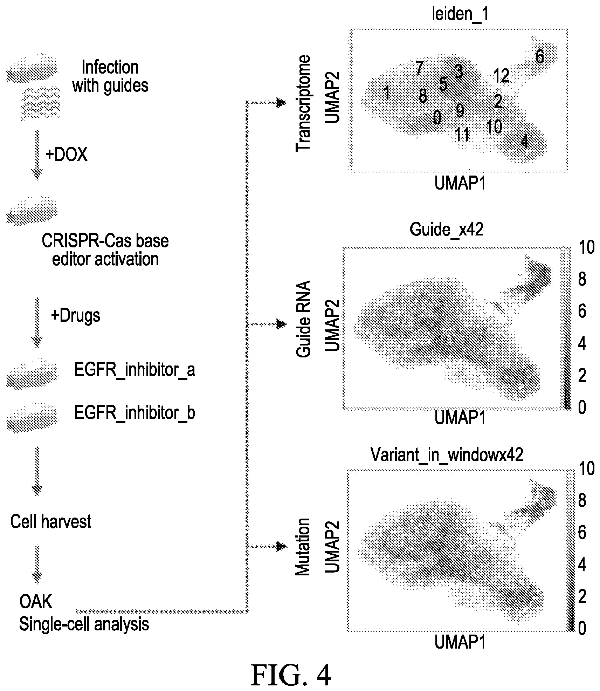

The cells can be subjected to gene modulation or regulation. For example, but not by way of limitation, cells for use in the present disclosure can be subjected to gene modulation or regulation by an enzyme or fusion protein that includes such an enzyme, *e.g*., by contacting the cells with the enzyme or fusion protein. The enzyme or fusion protein including such an enzyme can cut a target gene, nick a target gene, edit a target gene, repress expression of a target gene and/or activate expression of a target gene of a cell. The enzyme or fusion protein including such an enzyme is directed to a region of a target gene by a guide nucleic acid, *e.g.,* to modulate or regulate the target gene. Gene modulation or regulation is achieved by the nicking, cutting, editing, repression and/or activation of a target gene by an enzyme or a fusion protein that includes such an enzyme. Non-limiting examples of enzymes that can be used to modulate or regulate genes, *e.g*., for use in a gene editing system, include homing endonucleases or meganucleases, zinc finger nucleases (ZFN), transcription activator-like effector nucleases (TALENs), CRISPR enzymes and fusion proteins including such enzymes. Cells for use in the present disclosure can be subjected to a genome screen, *e.g*., where a target gene is modulated or regulated using an enzyme disclosed herein or a fusion protein including such an enzyme. A method of the present disclosure can be used in combination with single-cell genome screens, *e.g*., in combination with single-cell CRISPR-based screens as shown in FIG. 4. Non-limiting examples of CRISPR-based screens include gene editing CRISPR screens, CRISPR interference (CRISPRi) screens and CRISPR activation (CRISPRa) screens. For example, but not by way of limitation, a single-cell genome screen can be performed followed by a combinatorial indexing method of the present disclosure. Nucleic acids (*e.g.,* guide nucleic acid molecules (guide RNAs (gRNAs)) and other nucleic acids) associated with performing the single-cell genome screen *(e.g.,* a single-cell CRISPR-based screen) present in the genetically modified cells are processed through the combinatorial indexing method described herein. The indexed guide nucleic acid molecules (*e.g*., gRNAs) can further go through a secondary enrichment process before sequencing. In certain embodiments, the identity of the guide nucleic acid molecules (*e.g*., gRNAs) can be determined by either direct sequencing of the guide nucleic acid molecules (*e.g*., gRNAs) or by sequencing the surrogate barcode sequences associated with the guide nucleic acid molecules (*e.g*., gRNAs). A method that includes the combinatorial indexing method described herein and a single-cell genome screen, *e.g*., a single-cell CRISPR-based screen, can further include modifying and/or manipulating the cells prior to and/or during the single-cell genome screen. A method that includes the combinatorial indexing method described herein and a single-cell genome screen can further include treating the cells with one or more therapeutic agents (*e.g.,* drugs of interest), *e.g.,* prior to performing the single-cell genome screen. For example, but not by way of limitation, as shown in FIG. 4, a method that includes the combinatorial indexing method described herein and a single-cell CRISPR-based screen can further include treating the cells with one or more drugs of interest, *e.g*., prior to performing the single-cell CRISPR-based screen. A method that includes the combinatorial indexing method described herein and a single-cell genome screen, *e.g.,* a single-cell CRISPR-based screen, can further include subjecting the cells to development and/or differentiationInformation, *e.g.,* the sequence of a guide nucleic acid molecule (*e.g.,* a gRNA) and the associated cellular outcome (*e.g.,* editing, repression or activation of a gene and/or cell phenotype), obtained from a method that includes the combinatorial indexing method described herein and a single-cell genome screen can be used to determine the function of genes (*e.g*., genes targeted in the screen), responsiveness to drugs and/or mechanisms of drug resistance. A method of the present disclosure includes performing a single-cell genome screen followed by a combinatorial indexing method described herein.

Cells for use in the present disclosure can be genetically modified with a gene editing system. Non-limiting examples of gene editing systems include homing endonucleases or meganucleases, ZFN, TALENs and CRISPR gene editing systems. For example, but not by way of limitation, the methods of the present disclosure can be used to identify and/or quantity the nucleic acid modifications generated by the gene editing system.

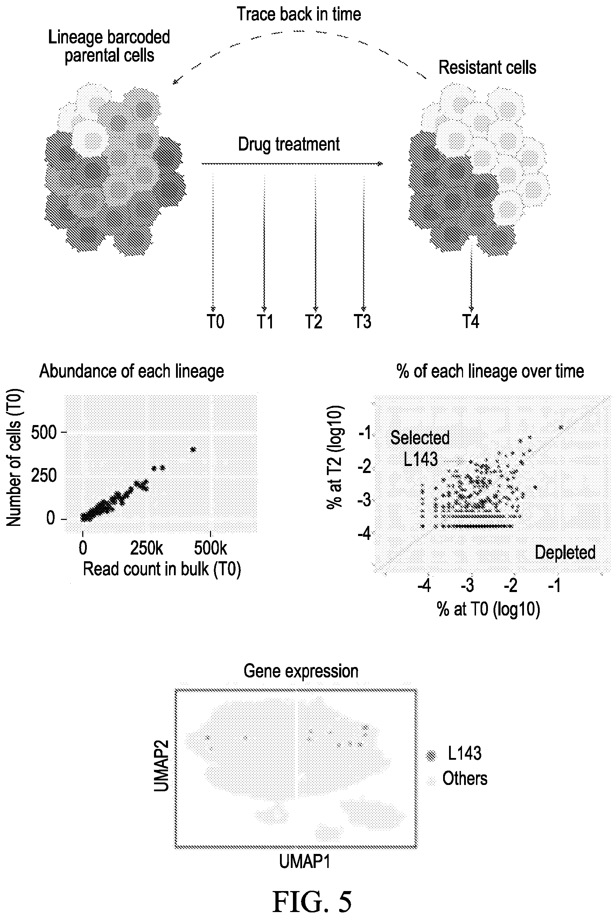

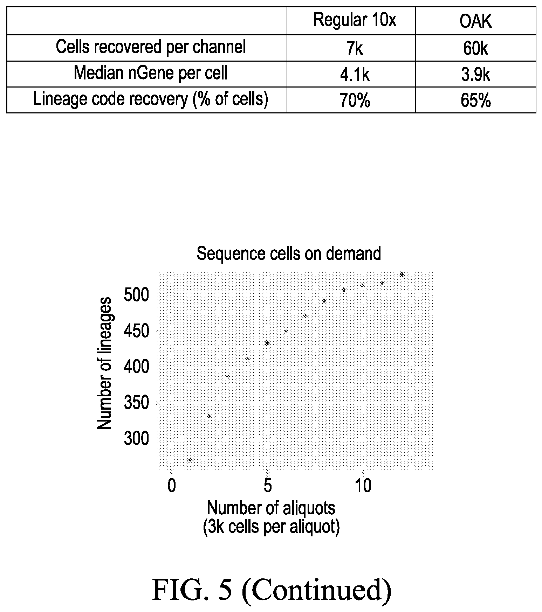

The cells can include one or more lineage barcodes. For example, but not by way of limitation, combinatorial indexing methods of the present disclosure using cells that have lineage barcodes can be performed in combination with single-cell lineage tracing techniques. The single-cell lineage tracing technique can be used to obtain information about lineage identity and to relate cells across multiple time points during development, differentiation and/or artificial manipulation (*e.g*., drug treatment). A method that includes the combinatorial indexing method described herein and a single-cell lineage tracing technique can be used to determine the type of cell, the genome of the cell or a specific genetic mutation associated with a cell that results in resistance to a drug of interest as shown in FIG. 5. A non-limiting example of a single-cell lineage tracing technique is TraCe-seq as disclosed in WO 2021/188973 and Chang et al., Nature Biotechnology 40:86-93 (2022). The lineage barcodes can be included in exogenous nucleic acid constructs that are introduced into the cells, *e.g.,* as described in Section III. Alternatively, the lineage barcodes can be endogenous genetic markers. The lineage barcodes and other nucleic acids associated with performing the single-cell lineage tracing technique present in the cells are processed through the combinatorial indexing method described herein. The lineage barcodes can further go through a secondary enrichment process before sequencing. A method of the present disclosure includes performing a lineage tracing technique and performing a combinatorial indexing method described herein at different timepoints during treatment of a cell population with a drug of interest. A method of the present disclosure includes performing a lineage tracing technique and performing a combinatorial indexing method described herein at different timepoints during the development and/or differentiation of cell population. Information obtained from a method that includes the combinatorial indexing method described herein and a single-cell lineage tracing technique can be used, *e.g*., information of lineage identity and the molecular phenotypes at a cellular level, can be used to determine how different lineages undergo diversified processes of development and/or differentiation. Information obtained from a method that includes the combinatorial indexing method described herein and a single-cell lineage tracing technique can be used to determine how certain lineages share similar programs in development and/or differentiation. Information obtained from a method that includes the combinatorial indexing method described herein and a single-cell lineage tracing technique can be used to determine how pre-existing genetic programs in each cell and lineage affect their response to external stimuli including, but not limited, to drug treatment, *e.g.*, as shown in FIG. 5.

The cells can include one or more hashtags. For example, but not by way of limitation, combinatorial indexing methods of the present disclosure can use cells that are bound by protein binding reagents coupled to an oligonucleotide comprising a barcode. The protein binding reagents coupled to an oligonucleotide comprising a barcode can be bound to the cells for use in the combinatorial indexing methods of the present disclosure as described in Section III. A method of the present disclosure includes performing a cell hashing technique and performing a combinatorial indexing method described herein. A non-limiting example of a cell hashing technique is CITE-seq, *e.g*., as disclosed in Stoeckius et al., Nature Methods 14:865-868 (2017). The barcodes coupled to the protein binding reagents and other nucleic acids associated with performing the cell hashing technique are processed through the combinatorial indexing method described herein.

The cells can be bacterial cells. For example, but not by way of limitation, the methods of the present disclosure can be used to analyze the microbiome of a subject, *e.g*., the gut microbiome of a subject.

The cells can include cells having different developmental stages. In certain embodiments, the cells can include cells of different disease states. For example, but not by way of limitation, the methods of the present disclosure can be used to analyze the genetic regulation of different developmental stages. The methods of the present disclosure can be used to analyze the molecular dynamics of the different cell types during distinct development stages. The methods of the present disclosure can be used for the identification of rare and/or transient developmental stages and/or disease states.

The cells can be from any model organism. For example, but not by way of limitation, the model organism can be *E*. *coli,* yeast, Arabidopsis, xenopus, zebrafish, drosophila melanogaster, ascidians, nematodes, mice and monkeys.

The cells can be cells that have been infected by an infectious agent. Non-limiting examples of infectious agents include viruses, bacteria, fungi and protozoans.

The cells can be enriched for cells of interest to produce an enriched cell sample, which can be subjected to the methods of the present disclosure. Any technique known in the art can be used to enrich for the cells of interest.

The nuclei can be isolated from any cell type. For example, but not by way of limitation, the nuclei can be isolated from any one of the cells disclosed herein. Methods for isolating nuclei from cells are known to the person skilled in the art and can be used to isolate the nuclei for use in the present disclosure.

### III. METHODS OF USE

The present disclosure relates to improved methods for combinatorial indexing of nucleic acids from single cells or nuclei. Methods of the present disclosure comprise the incorporation of at least two index sequences into the nucleic acids of a single cell or nuclei. FIG. 1 provides a flowchart of an exemplary method of the present disclosure.

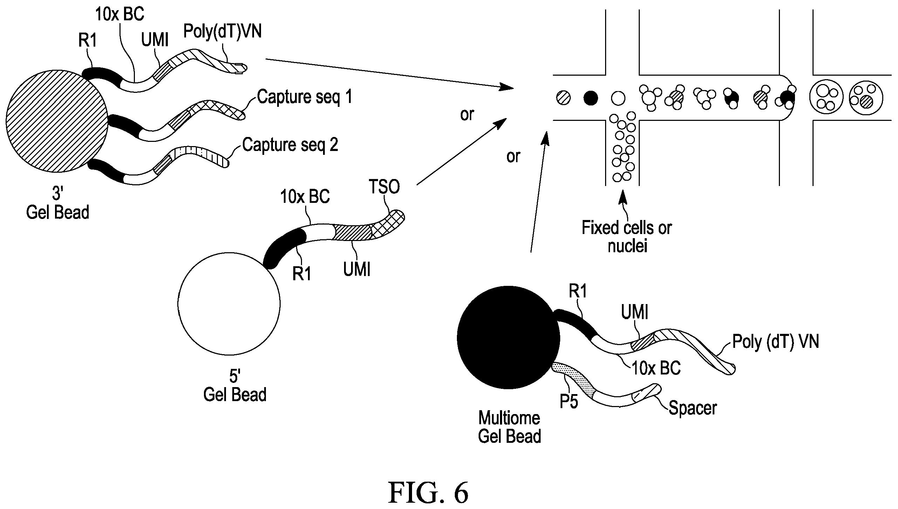

A method of the present disclosure can comprise contacting cells or nuclei isolated from cells with particles that comprise a first index sequence. FIG. 6 provides a schematic showing exemplary particles, *e.g*., gel beads, that can be used in the present disclosure. In a method of the present disclosure contacting cells or nuclei with particles can comprise contacting the cells or nuclei with the particles to produce a mixture of the cells or nuclei and the particles. For example, but not by way of limitation, the cells or nuclei are present in an aqueous phase and the particles are present in a second aqueous phase, and the two aqueous phases are combined to produce a single aqueous phase of cells or nuclei and particles.

In a method of the present disclosure a plurality of cells or nuclei isolated from a plurality of cells are contacted with the particles. A plurality of cells can include at least about 5,000 cells. For example, but not by way of limitation, the plurality of cells includes about 10,000 or more cells, about 20,000 or more cells, about 30,000 or more cells, about 40,000 or more cells, about 50,000 or more cells, about 100,000 or more cells, about 150,000 or more cells, about 200,000 or more cells, about 300,000 or more cells, about 400,000 or more cells or 500,000 or more cells. A plurality of cells includes at least about 100,000 cells. A plurality of cells includes at least about 200,000 cells. A plurality of cells includes from about 5,000 to about 200,000 cells, e.g., from about 10,000 to about 200,000 cells, from about 50,000 to about 200,000 cells, from about 100,000 to about 200,000 cells, from about 150,000 to about 200,000 cells, from about 5,000 to about 150,000 cells, from about 5,000 to about 100,000 cells, from about 5,000 to about 50,000 cells, from about 5,000 to about 10,000 cells, from about 10,000 to about 150,000 cells or from about 50,000 to about 100,000 cells.

In a method of the present disclosure, a plurality of particles can be contacted with the cells or nuclei, *e.g.,* the plurality of cells or nuclei isolated from a plurality of cells. A plurality of particles, *e.g*., beads, include about 5,000 to about 1,000,000 particles. For example, but not by way of limitation, the plurality of particles includes about 10,000 to about 1,000,000 particles, about 20,000 to about 1,000,000 particles, about 30,000 to about 1,000,000 particles, about 40,000 to about 1,000,000 particles, about 50,000 to about 1,000,000 particles, about 60,000 to about 1,000,000 particles, about 70,000 to about 1,000,000 particles, about 80,000 to about 1,000,000 particles, about 90,000 to about 1,000,000 particles, about 100,000 to about 1,000,000 particles, about 200,000 to about 1,000,000 particles, about 300,000 to about 1,000,000 particles, about 400,000 to about 1,000,000 particles, about 500,000 to about 1,000,000 particles, about 600,000 to about 1,000,000 particles, about 700,000 to about 1,000,000 particles, about 800,000 to about 1,000,000 particles, about 900,000 to about 1,000,000 particles, about 10,000 to about 500,000 particles, about 20,000 to about 500,000 particles, about 30,000 to about 500,000 particles, about 40,000 to about 500,000 particles, about 50,000 to about 500,000 particles, about 60,000 to about 500,000 particles, about 70,000 to about 500,000 particles, about 80,000 to about 500,000 particles, about 90,000 to about 500,000 particles, about 100,000 to about 500,000 particles, about 200,000 to about 500,000 particles, about 300,000 to about 500,000 particles, about 400,000 to about 500,000 particles, about 10,000 to about 400,000 particles, about 10,000 to about 300,000 particles, about 10,000 to about 200,000 particles, about 10,000 to about 100,000 particles, about 50,000 to about 200,000 particles, about 60,000 to about 190,000 particles, about 70,000 to about 180,000 particles, about 80,000 to about 170,000 particles, about 90,000 to about 150,000 particles or about 90,000 to about 110,000 particles. The plurality of particles includes about 50,000 to about 450,000 particles. The plurality of particles includes about 400,000 to about 1,000,000 particles. The plurality of particles includes about 200,000 to about 800,000 particles. In a method of the present disclosure the plurality of particles includes about 300,000 to about 800,000 particles. The plurality of particles includes about 400,000 to about 800,000 particles. In a method of the present disclosure the plurality of particles includes about 50,000 particles. In a method of the present disclosure the plurality of particles includes about 100,000 particles. In a method of the present disclosure the plurality of particles includes about 400,000 particles.

In a method of the present disclosure, the particles that are combined with the cells or nuclei can be glass, plastic, gel or metal particles. The particles can be beads, *e.g*., microbeads. In a method of the present disclosure, the particles are gel beads, *e.g*., gel microbeads. The particles can be any shape, *e.g.,* the particles can have a spherical, non-spherical, oval, oblong, amorphous, circular or cylindrical shape. In a method of the present disclosure, the particles can have a diameter of about 0.5 to about 500 µm. In a method of the present disclosure, the particles have a diameter less than about 0.5 µm, less than about 1.0 µm, less than about 1.5 µm, less than about 2.0 µm, less than about 5.0 µm, less than about 10.0 µm, less than about 20.0 µm, less than about 30.0 µm, less than about 40.0 µm, less than about 50.0 µm, less than about 60 µm, less than about 70 µm, less than about 80 µm, less than about 90 µm, less than about 100 µm, less than about 250 µm or less than about 500 µm.

In a method of the present disclosure, the first index sequence is coupled to the particles, *e.g.,* beads. The first index sequence ban be coupled to the particles, *e.g.,* beads, using a linker. In a method of the present disclosure, the first index sequence is releasably attached to the particles, *e.g*., beads. In a method of the present disclosure, each particle can have multiple index sequences attached, *e.g*., about 10 index sequences, about 100 index sequences, about 1,000 index sequences, about 10,000 index sequences or about 100,000 index sequences or more. In a method of the present disclosure, each particle can have from about 10 to about 100,000 index sequences attached, *e.g*., from about 50 to about 100,000, from about 100 to about 100,000, from about 1,000 to about 100,000, from about 10,000 to about 100,000, from about 50,000 to about 100,000, from about 10 to about 50,000, from about 10 to about 10,000, from about 10 to about 1,000, from about 10 to about 100, from about 10 to about 50, from about 100 to about 50,000, from about 1,000 to about 50,000, from about 100 to about 10,000 or from about 1,000 to about 100,000 index sequences attached. , All of the index sequences can be attached to a particular particle, *e.g*., bead, can have the same nucleotide sequence. In a method of the present disclosure, all of the index sequences attached to a particular particle, *e.g*., bead, can have the same nucleotide sequence, and a large number of diverse index sequences can be represented across the plurality of particles, *e.g*., beads, used. For example, but not by way of limitation, the plurality of beads can include a diverse index sequence library that includes at least about 100 different index sequences, at least about 1,000 different index sequences, at least about 10,000 different index sequences, at least about 100,000 index sequences, at least about 1,000,000 different index sequences, at least about 1,500,000 different index sequences, at least about 2,000,000 different index sequences, at least about 2,500,000 different index sequences, at least about 3,000,000 different index sequences, at least about 3,500,000 different index sequences, at least about 4,000,000 different index sequences, at least about 4,500,000 different index sequences or at least about 5,000,000 different index sequences. In a method of the present disclosure, the plurality of beads can include from about 100 to about 5,000,000 different index sequences, *e.g*., from about 100 to about 1,000,000, about 100 to about 500,000, about 100 to about 50,000, about 100 to about 10,000, about 100 to about 1,000, about 100 to about 500, about 500 to about 5,000,000, about 1,000 to about 5,000,000, about 10,000 to about 5,000,000, about 50,000 to about 5,000,000, about 500,000 to about 5,000,000, about 1,000,000 to about 5,000,000, about 10,000 to about 1,000,000, about 100,000 to about 1,000,000 or about 500,000 to about 1,000,000 different index sequences.

In a method of the present disclosure, the first index sequence of each particle, *e.g*., bead, is unique relative to other particles in the plurality of particles. For example, but not by way of limitation, the first index sequence attached to a particle present in an emulsion droplet is unique compared to the sequence of a first index sequence attached to a particle present in a different emulsion droplet. Alternatively, no more than about 50% of the particle population in the plurality of particles, *e.g*., no more than about 45%, no more than about 40%, no more than about 35%, no more than about 30%, no more than about 25%, no more than about 20%, no more than about 15%, no more than about 10%, no more than about 9%, no more than about 8%, no more than about 7%, no more than about 6%, no more than about 5%, no more than about 4%, no more than about 3%, no more than about 2% or no more than about 1% of the particle population, are attached to an index sequence having the same nucleic acid sequence as a different particle. In a method of the present disclosure, no more than about 10% of the particle population in the plurality of particles are attached to an index sequence having the same nucleic acid sequence as a different particle. In a method of the present disclosure, no more than about 5% of the particle population in the plurality of particles are attached to an index sequence having the same nucleic acid sequence as a different particle. In a method of the present disclosure, from about 1% to about 50% of the particle population in the plurality of particles (*e.g*., from about 1% to about 40%, from about 1% to about 30%, from about 1% to about 20%, from about 1% to about 10%, from about 1% to about 5%, from about 5% to about 50%, from about 10% to about 50%, from about 20% to about 50%, from about 30% to about 50%, from about 40% to about 50%, from about 5% to about 40%, from about 5% to about 20% or from about 5% to about 10%) are attached to an index sequence having the same nucleic acid sequence as a different particle.

In a method of the present disclosure, an index sequence for use in the present disclosure, *e.g.,* a first index sequence and/or a second index sequence, can be up to 200 nucleotides in length. In a method of the present disclosure, the index sequence can be about 5 to about 200 nucleotides in length, *e.g*., about 5 to about 150 nucleotides in length, about 5 to about 100 nucleotides in length, about 5 to about 50 nucleotides in length, about 10 to about 150 nucleotides in length, about 20 to about 100 nucleotides in length or about 10 to about 100 nucleotides in length. The index sequence can be about 5 to about 50 nucleotides in length, e.g., about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 50 nucleotides in length, about 15 to about 50 nucleotides in length, about 20 to about 50 nucleotides in length, about 25 to about 50 nucleotides in length, about 30 to about 50 nucleotides in length, about 35 to about 50 nucleotides in length, about 40 to about 50 nucleotides in length, about 10 to about 40 nucleotides in length or about 10 to about 30 nucleotides in length.

In a method of the present disclosure, the first index sequence is provided in a first polynucleotide. For example, but not by way of limitation, the first polypeptide that comprises the first index sequence is coupled to the particles, *e.g*., beads, as described herein. In a method of the present disclosure, the first polynucleotide can further comprise a universal sequence. For example, but not by way of limitation, the universal sequence can be a sequence that is common to two or more first polynucleotides. In a method of the present disclosure, the universal sequence can be a sequence that is common for all of the first polynucleotides. In a method of the present disclosure, the universal sequence can be used as a primer binding site, *e.g.,* for binding a primer to be used in an amplification process, or function as a primer. In a method of the present disclosure, the first polynucleotide can further comprise a sequence that is at least partially complementary to a sequence of a nucleic acid of the cells or nuclei. In a method of the present disclosure, the universal sequence can include a sequence that binds to RNA poly A tail. For example, but not by way of limitation, the universal sequence can include a poly T sequence that binds to the poly A tails of mRNAs. In a method of the present disclosure, the first polynucleotide comprises a universal sequence and a first index sequence. In a method of the present disclosure, the first polynucleotide comprises a first index sequence and a sequence complementary to a sequence of a nucleic acid of the cells or nuclei. In a method of the present disclosure, the first polynucleotide comprises a universal sequence, a first index sequence and a sequence complementary to a sequence of a nucleic acid of the cells or nuclei. In a method of the present disclosure, the first polynucleotide comprises a universal sequence, a first index sequence to identify the partition (*e.g*., emulsion droplet) and additional sequences, for example, but not by way of limitation, unique molecular identifiers.

In a method of the present disclosure, the universal sequence can be designed to capture chromatin fragments and any other DNA fragments that result from transposome treatment. Non-limiting examples of such a capture mechanism can include binding of complementary sequences and ligation. In a method of the present disclosure, the universal sequence can be designed to capture nucleic acids that are introduced to the cells or nuclei by incubation, electroporation, infection, editing or other methods. Non-limiting examples of such a capture mechanism include binding of complementary sequences and ligation. The introduced nucleic acids can include, but are not limited to, guide RNAs (gRNAs), lineage barcodes, oligonucleotides conjugated with antibodies, cell hashing oligonucleotides and other oligonucleotides used for sample multiplexing.

In a method of the present disclosure, the first polynucleotide can exist in multiple forms and use multiple forms of universal sequences to allow simultaneous capture of multiple molecular types. For example, but not by way of limitation, the molecular types can be RNA, DNA and synthetic oligonucleotides that are associated with antibodies, peptides, proteins, molecule complexes, lipids or cholesterol. In a method of the present disclosure, the first polynucleotide includes additional universal sequences that assist the integration of the second index sequence.

In a method of the present disclosure, the cells and/or nuclei can be fixed prior to being used in the methods of the present disclosure. For example, but not by way of limitation, the cells or nuclei are fixed prior to contacting the cells or nuclei with the particles coupled to the first index sequence. Any technique for fixation of cells and/or nuclei known in the art can be used in the present disclosure. For example, but not by way of limitation, the cells and/or nucleic can be contacted with a fixative. In a method of the present disclosure, the fixation process can comprise chemical cross-linking, *e.g*., by using glutaraldehyde and/or formaldehyde. The cells or nuclei can be fixed with an alcohol, *e.g*., methanol and/or ethanol.

The cells can be permeabilized prior to being used in the methods of the present disclosure. For example, but not by way of limitation, the cells are permeabilized prior to contacting the cells or nuclei with the particles coupled to the first index sequence. Any technique for permeabilizing cells known in the art can be used in the present disclosure. For example, but not by way of limitation, the cells can be permeabilized with an alcohol, *e.g.,* methanol, and/or a detergent, *e.g.,* such as Tween-20. In certain embodiments, the cells can be fixed and permeabilized.

In a method of the present disclosure, the cells, *e.g.,* cells of a tissue, can be dissociated prior to use in the methods of the present disclosure. For example, but not by way of limitation, the cells can be dissociated to generate a single cell suspension for use in the present disclosure. In a method of the present disclosure, the cells can be dissociated prior to contacting the cells (or nuclei obtained from such cells) with the particles coupled to the first index sequence. Methods for dissociating cells, *e.g.,* of a tissue, are known in the art.

In a method of the present disclosure, the cells or nuclei can be crosslinked prior to use in the methods of the present disclosure. For example, but not by way of limitation, the cells or nuclei can be crosslinked prior to contacting the cells or nuclei with the particles coupled to the first index sequence. Alternatively or additionally, the cells or nuclei can be crosslinked in the emulsion droplet. In a method of the present disclosure, the membranes of the cells for use in the present disclosure can be crosslinked. Crosslinking of the cells, *e.g*., crosslinking the membranes of the cells, or nuclei can be achieved by exposing the cells to any crosslinking agent. In a method of the present disclosure, crosslinking can be performed using a fixative, as disclosed herein. In a method of the present disclosure, cross-linking can be performed by exposing the cells to dithiobis(succinimidyl propionate) (DSP).

The nuclei for use in the present disclosure can be nucleosome free. The nuclei can be treated to deplete the nuclei of nucleosomes. The nuclei can be treated to deplete the nuclei of nucleosomes prior to be used in any one of the disclosed methods. For example, but not by way of limitation, the nuclei can be treated to deplete the nuclei of nucleosomes prior to contacting the nuclei with the particles coupled to the first index sequence. Non-limiting examples of methods for depleting nuclei of nucleosomes comprise contacting isolated nuclei with a chaotropic agent or a detergent capable of disrupting the interactions between nucleic acids and proteins. Nucleosome-free or depleted nuclei can be used in the methods of the present disclosure to index genomic DNA.

The cells or nuclei for use in the present disclosure can be contacted with a transposase. Non-limiting examples of transposases include Tn5, Mu and Tn7 or mutants thereof. For example, but not by way of limitation, the transposase can be a hyperactive mutation of the transposase, *e.g*., a hyperactive Tn5 transposase. Non-limiting examples of hyperactive Tn5 variants are disclosed in Picelli et al., Genome Res. 24(12):2033-2040 (2014), the contents of which are disclosed herein in its entirety. In certain embodiments, the nuclei can be treated with a transposase or transposome (*e.g*., a transposase in complex with DNA including transposase recognition sequences) prior to use in the present disclosure. For example, but not by way of limitation, the nuclei can be treated with a transposase or transposome prior to contacting the nuclei with the particles coupled to the first index sequence. Alternatively or additionally, the transposase or transposome can be partitioned into the emulsion droplet with the particle and the two or more nuclei. In a method of the present disclosure, the transposome is in complex with a DNA sequence that comprises a transposon flanked by transposase recognition sequences, where the transposon is inserted into genomic DNA. In a method of the present disclosure, the transposon can comprise primer binding sequences and/or adaptors. In a method of the present disclosure, treating nuclei with a transposase or transposome can be used in the methods of the present disclosure for Assay for Transposase-Accessible Chromatin (ATAC) sequencing.

The method can further comprise partitioning individual particles and one or more of the cells or nuclei into a compartment. In certain embodiments, each compartment comprises two or more cells or nuclei. In certain embodiments, the compartment is an emulsion. For example, but not by way of limitation, the compartment is an emulsion droplet.

In a method of the present disclosure, the compartment is not a well of a multi-well plate, *e.g.,* a well of a microtiter plate.

In a method of the present disclosure, individual beads and one or more of the cells or nuclei can be partitioned into emulsion droplets. In a method of the present disclosure, partitioning the individual particles and the one or more cells or nuclei into emulsion droplets can comprise bringing the plurality of particles from a first aqueous phase and the cells or nuclei from a second aqueous phase in contact with another phase, *e.g.,* a non-aqueous phase, that is immiscible with the first and/or second aqueous phase to form emulsion droplets. Alternatively, partitioning the individual particles and the one or more cells or nuclei into emulsion droplets can comprise bringing a first aqueous phase comprising the plurality of particles and the cells or nuclei in contact with another phase, *e.g*., a non-aqueous phase, that is immiscible with the first aqueous phase to form emulsion droplets.

In a method of the present disclosure, the emulsion droplets can be generated by providing the cells and/or nucleic in a first fluid, *e.g.,* a first aqueous phase, and combining the first fluid with a second fluid, *e.g*., a second aqueous phase or a non-aqueous phase, and shearing the fluids to generate a plurality of emulsion droplets that contain an individual particle and one or more of the cells or nuclei. In a method of the present disclosure, shearing the fluids can be performed using any known method, technique or device for mixing solutions. For example, but not by way of limitation, shearing the fluids can include vortexing, shaking, flicking, stirring and/or pipetting. In a method of the present disclosure, shearing the fluids includes vortexing the fluids to generate emulsion droplets.

In a method of the present disclosure, the emulsion droplets can be generated using any emulsion droplet generating device. In a method of the present disclosure, the emulsion droplet generating device can be a microfluidic device. For example, but not by way of limitation, droplets can be generated on a Chromium^{™} platform commercialized by 10xGenomics. In a method of the present disclosure, a first aqueous phase comprising the index sequence carrying beads is flowed through a channel segment and a second aqueous phase comprising the cells or nuclei is flowed through a second channel segment towards a channel junction. A partitioning fluid (*e.g.,* oil) is introduced into the channel junction from one or more side channels, and the combined streams are flowed into an outlet channel, as shown in FIG. 1. Within the channel junction, the two aqueous streams are combined with the partitioning oil, and partitioned into droplets that contain co-partitioned nucleic or cells and beads into emulsion droplets (referred to herein as "GEMs") (FIG. 1). In a method of the present disclosure, controlling the flow characteristics of each of the fluids combining at the channel junction and controlling the geometry of the channel junction, one can achieve a desired occupancy level of beads, cells (or nuclei) or both, within the droplets that are generated. Additional disclosure regarding microfluidic devices for use in the present disclosure is provided in WO 2017/096158 and U.S. Patent No. 11,193,122, the contents of each which are incorporated herein in their entirety.

In a method of the present disclosure, partitioning the individual particles and the one or more cells or nuclei can result in the generation of a plurality of emulsion droplets. In a method of the present disclosure, the methods of the present disclosure can comprise the generation of at least about 1,000 droplets, at least about 2,000 droplets, at least about 3,000 droplets, at least about 4,000 droplets, at least about 5,000 droplets, at least about 6,000 droplets, at least about 7,000 droplets, at least about 8,000 droplets, at least about 9,000 droplets, at least about 10,000 droplets, at least about 20,000 droplets, at least about 30,000 droplets, at least about 40,000 droplets, at least about 50,000 droplets, at least about 60,000 droplets, at least about 70,000 droplets, at least about 80,000 droplets, at least about 90,000 droplets, at least about 100,000 droplets, at least about 200,000 droplets, at least about 300,000 droplets, at least about 400,000 droplets, at least about 500,000 droplets, at least about 600,000 droplets, at least about 700,000 droplets, at least about 800,000 droplets, at least about 900,000 droplets or at least about 1,000,000 droplets. In a method of the present disclosure, from about 5,000 to about 200,000 droplets are generated.

In a method of the present disclosure, each compartment, *e.g*., droplet, comprises more than one cell or nuclei along with a single bead. For example, but not by way of limitation, the number of cells or nuclei in each droplet can be from about 2 to about 20. The number of cells or nuclei in each droplet can be from about 3 to about 20, from about 4 to about 20, from about 5 to about 20, from about 6 to about 20, from about 7 to about 20, from about 8 to about 20, from about 9 to about 20, from about 10 to about 20, from about 11 to about 20, from about 12 to about 20, from about 13 to about 20, from about 14 to about 20, from about 15 to about 20, from about 16 to about 20, from about 17 to about 20, from about 18 to about 20, from about 19 to about 20, from about 2 to about 19, from about 2 to about 18, from about 2 to about 17, from about 2 to about 16, from about 2 to about 15, from about 2 to about 14, from about 2 to about 13, from about 2 to about 12, from about 2 to about 11, from about 2 to about 10, from about 2 to about 9, from about 2 to about 8, from about 2 to about 7, from about 2 to about 6, from about 2 to about 5, from about 2 to about 4, from about 2 to about 3 or from about 3 to about 10 cells or nuclei. In certain embodiments, each droplet contains at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19 or at least about 20 cells or nuclei. The number of cells or nuclei in each droplet can be from about 2 to about 10. In a method of the present disclosure, from about 5,000 to about 200,000 droplets are generated and each droplet can include from about 2 to about 10 cells or nuclei.

The incorporation of more than one cell or nuclei in each emulsion droplet along with a single bead allows the indexing of the nucleic acids of the cells or nuclei present in the emulsion droplet with the same index sequence. As described herein, the incorporation of more than one cell or nuclei in each emulsion droplet increases the throughput of the indexing technique and increases the capacity of the emulsion droplet generating device, *e.g*., microfluidic device, being used to generate the droplets by at least 20-fold.

In a method of the present disclosure, other reagents can be co-partitioned into the emulsion droplets. In a method of the present disclosure, such reagents can be used to incorporate the first index sequence into the nucleic acids, *e.g.,* for use in the amplification process or ligation process for incorporating the first index sequence into the nucleic acids. For example, but not by way of limitation, the reagents can comprise polymerases, reverse transcriptases, nucleoside triphosphates or NTP analogues, primers, cofactors, ligation reaction reagents, endonucleases, lysis reagents, dyes, markers or labels. In a method of the present disclosure, additional reagents can include proteases to remove proteins bound to the nucleic acids of the cell or nuclei and/or transposases or transposomes to fragment or insert a known sequence into the nucleic acids of a cell or nuclei.

The method can further comprise incorporating the first index sequence disposed upon the particle, *e.g.,* bead, into the nucleic acids of the cells or nuclei to generate indexed nucleic acids. In a method of the present disclosure, incorporating the first index sequence into the nucleic acids occurs within the droplet, *e.g.,* using the reagents that are co-partitioned into the droplets. Any suitable nucleic acid amplification method known in the art can be used to incorporate the first index sequence into the nucleic acids of the cells or nuclei. Non-limiting examples of such amplification methods include polymerase chain reaction (PCR), reverse transcriptase PCR, real-time PCR, rolling circle amplification (RCA), self-sustained sequence replication (3SR), nucleic acid sequence-based amplification (NASBA), strand displacement amplification (SDA), transcription-mediated amplification (TMA), single primer isothermal amplification (SPIA), helicase-dependent amplification (HDA), loop mediated amplification (LAMP), recombinase-polymerase amplification (RPA), nicking enzyme amplification reaction (NEAR), nicking endonuclease assisted nanoparticle activation (NENNA) and ligase chain reaction (LCR). In a method of the present disclosure, the amplification process is an isothermal amplification process, *e.g.*, TMA, NEAR and/or RPA. Fakruddin et al., J. Pharm. Bioallied. Sci. 5(4): 245-252 (2013) and Yan et al., Mol. BioSyst. 10:970-1003 (2014) disclose additional amplification methods for use in the present disclosure, the contents of each of which are disclosed in their entireties herein. In a method of the present disclosure, the amplification process uses the universal sequence, *e.g.*, present in the first polynucleotide, for binding the primers used in the amplification process. Alternatively, a ligation process can be used to incorporate the first index sequence into the nucleic acids of the cells or nuclei. For example, but not by way of limitation, the index sequence can be ligated to the nucleic acids of the single cells or nuclei by a ligase.

In a method of the present disclosure, reverse transcription is used to incorporate the first index sequence into the nucleic acids of the cells or nuclei. For example, but not by way of limitation, reverse transcription is used to incorporate the first index sequence into RNA of the cells, *e.g.*, mRNA of the cells. In a method of the present disclosure, within the compartment, *e.g.*, emulsion droplet, the first polynucleotide providing the first index sequence binds to an RNA, *e.g.*, mRNA, of the cell. A reverse transcriptase binds to the RNA complexed with first polynucleotide and initiates the synthesis of a complementary DNA (cDNA) strand. Any reverse transcriptase known in the art can be used in the method of the present disclosure. In a method of the present disclosure, RNase H can be present within the partition, e.g., emulsion droplet, to degrade the RNA of the cDNA:RNA complex. The reverse transcriptase can lack RNase H activity. The reverse transcriptase can have the terminal transferase activity and the template-switching activity to incorporate a universal sequence of choice at the end of the cDNA strand. The compartment, *e.g*., emulsion droplet, can further comprise a DNA-dependent polymerase that uses the single-stranded cDNA as a template to synthesize a complementary cDNA strand to form double-stranded cDNA.

In a method of the present disclosure, PCR is used to incorporate the first index sequence into the nucleic acids of the cells or nuclei. For example, but not by way of limitation, PCR is used if the nucleic acid that is to be indexed is DNA, *e.g*., genomic DNA.

In a method of the present disclosure, the first index sequences are released from the particles, *e.g*., gel beads. In a method of the present disclosure, the index sequences are released from the particles prior to the incorporation of the index sequences into the nucleic acids. Alternatively or additionally, the index sequences are released from the particles after the incorporation of the index sequences into the nucleic acids. In a method of the present disclosure, the first index sequences are released from the particles by dissolving the particles, *e.g.,* gel beads. The particles can be dissolved or degraded using a reducing agent, changing the temperature, *e.g*., heating the droplets, changing the pH and/or exposure to light. In a method of the present disclosure, the first index sequences are released from the particles by cleaving the linker that couples the index sequences to the particles. In a method of the present disclosure, the first index sequences are released from the particles within the droplet.

In a method of the present disclosure, the method can further comprise combining the indexed nucleic acids from a plurality of the compartments, *e.g*., emulsion droplets, to generate pooled indexed nucleic acids. For example, but not by way of limitation, the method includes combining cells and/or nuclei that comprise or are associated with the indexed nucleic acids from a plurality of the compartments, *e.g*., emulsion droplets, to generate pooled cells and/or nuclei. In a method of the present disclosure, the nucleic acids containing the first index sequence are removed from the compartment, *e.g*., emulsion droplet. For example, but not by way of limitation, the nucleic acids containing the first index sequence can be separated from the emulsion droplets by an unpacking process, where the indexed nucleic acids present in the emulsion droplets are released. Any technique known in the art for breaking an emulsion can be used for the unpacking process. Non-limiting examples of techniques used for breaking an emulsion include acidification, centrifugation, filtration and the addition of salt. In a method of the present disclosure, the cells containing the indexed nucleic acids remain intact during the incorporation of the first index sequence and the unpacking process releases the intact cells from the emulsion droplets.

In a method of the present disclosure, a subset of the released indexed nucleic acids is pooled together. In a method of the present disclosure, a subset of the cells or nuclei including the indexed nucleic acids released from the compartments, *e.g*., emulsion droplets, are pooled together. For example, but not by way of limitation, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% of the indexed nucleic acids (or cells comprising or associated with the indexed nucleic acids) released from the compartments, *e.g.*, emulsion droplets, are pooled together. In a method of the present disclosure, at least about 1% of the indexed nucleic acids (or cells comprising or associated with the indexed nucleic acids) released from the compartments, *e.g.*, emulsion droplets, are pooled together. In a method of the present disclosure, at least about 2% of the indexed nucleic acids (or cells comprising or associated with the indexed nucleic acids) released from the compartments, *e.g.*, emulsion droplets, are pooled together. In a method of the present disclosure, at least about 10% of the indexed nucleic acids (or cells comprising or associated with the indexed nucleic acids) released from the compartments, *e.g.*, emulsion droplets, are pooled together. In a method of the present disclosure, at least about 50% of the indexed nucleic acids (or cells comprising or associated with the indexed nucleic acids) released from the compartments, *e.g.*, emulsion droplets, are pooled together. In a method of the present disclosure, about 1% to about 5% of the indexed nucleic acids (or cells comprising or associated with the indexed nucleic acids) released from the compartments, *e.g.*, emulsion droplets, are pooled together. In a method of the present disclosure, about 1% to about 10% of the indexed nucleic acids (or cells comprising or associated with the indexed nucleic acids) released from the compartments, *e.g.*, emulsion droplets, are pooled together. In a method of the present disclosure, all the indexed nucleic acids released from the compartments, *e.g.*, emulsion droplets, are pooled together. In a method of the present disclosure, all of the cells including the indexed nucleic acids released from the compartments, *e.g.*, emulsion droplets, are pooled together.

The method can further comprise distributing subsets of the pooled indexed nucleic acids into a plurality of compartments comprising a second index sequence. The indexed cells or nuclei can be split into a plurality of compartments to acquire a second index sequence. Non-limiting examples of compartments include tubes, plates, vials and wells. In a method of the present disclosure, the compartment can be a well of a multi-well plate such as a 48-well, 96-well, a 384-well or 1536-well plate, *e.g.,* a well of a microtiter plate. In a method of the present disclosure, the compartment where a second index sequence is acquired by the indexed nucleic acids is not an emulsion droplet.

In a method of the present disclosure, each compartment, *e.g.*, well, contains more than one cell or nucleus comprising the indexed nucleic acids. For example, but not by way of limitation, the number of cells or nuclei in each compartment can be from about 2 to about 20,000. In a method of the present disclosure, the number of cells or nuclei in each compartment, *e.g.,* well, can be from about 10 to about 20,000, from about 100 to about 20,000, from about 1,000 to about 20,000, from about 2,000 to about 20,000, from about 3,000 to about 20,000, from about 4,000 to about 20,000, from about 5,000 to about 20,000, from about 6,000 to about 20,000, from about 7,000 to about 20,000, from about 8,000 to about 20,000, from about 9,000 to about 20,000, from about 10,000 to about 20,000, from about 11,000 to about 20,000, from about 12,000 to about 20,000, from about 13,000 to about 20,000, from about 14,000 to about 20,000, from about 15,000 to about 20,000, from about 16,000 to about 20,000, from about 17,000 to about 20,000, from about 18,000 to about 20,000, from about 19,000 to about 20,000, from about 2 to about 19,000, from about 2 to about 18,000, from about 2 to about 17,000, from about 2 to about 16,000, from about 2 to about 15,000, from about 2 to about 14,000, from about 2 to about 13,000, from about 2 to about 12,000, from about 2 to about 11,000, from about 2 to about 10,000, from about 2 to about 9,000, from about 2 to about 8,000, from about 2 to about 7,000, from about 2 to about 6,000, from about 2 to about 5,000, from about 2 to about 4,000, from about 2 to about 3,000, from about 2 to about 2,000, from about 2 to about 1,000 or from about 2 to about 500 cells or nuclei. In a method of the present disclosure, the number of cells or nuclei in each compartment, *e.g.,* well, can be from about 100 to about 15,000, from about 5,000 to about 15,000, from about 1,000 to about 15,000, from about 2,000 to about 15,000, from about 3,000 to about 15,000, from about 4,000 to about 15,000, from about 5,000 to about 15,000, from about 6,000 to about 15,000, from about 7,000 to about 15,000, from about 8,000 to about 15,000, from about 9,000 to about 15,000, from about 9,000 to about 12,000, from about 500 to about 10,000, from about 1,000 to about 10,000, from about 2,000 to about 10,000, from about 3,000 to about 10,000, from about 4,000 to about 10,000, from about 5,000 to about 10,000, from about 6,000 to about 10,000, from about 7,000 to about 10,000, from about 8,000 to about 10,000 or from about 9,000 to about 10,000. In a method of the present disclosure, each compartment contains at least about 2, at least about 10, at least about 100, at least about 500, at least about 1,000, at least about 2,000, at least about 3,000, at least about 4,000, at least about 5,000, at least about 6,000, at least about 7,000, at least about 8,000, at least about 9,000, at least about 10,000, at least about 11,000, at least about 12,000, at least about 13,000, at least about 14,000, at least about 15,000, at least about 16,000, at least about 17,000, at least about 18,000, at least about 19,000 or at least about 20,000 cells or nuclei. In a method of the present disclosure, the pooled indexed nucleic acids, *e.g.,* pooled cells or nuclei including the indexed nucleic acids, are sufficiently mixed so that cells or nuclei that have nucleic acids indexed with the same first index sequence are not distributed into the same compartment. As disclosed herein, the use of two index sequences in the methods of the present disclosure allows the labeling of nucleic acids from a single cell with a unique combination of the two index sequences.

The method can further comprise incorporating the second index sequence into the indexed nucleic acids in each compartment to generate dual-indexed nucleic acids. Any suitable nucleic acid sequence amplification method known in the art can be used to incorporate the second index sequence into the indexed nucleic acids of the cells or nuclei. Non-limiting examples of such amplification methods are provided herein. For example, but not by way of limitation, the amplification process is an isothermal amplification process. In a method of the present disclosure, the second index sequence can be incorporated into the indexed nucleic acids using a ligation process. In a method of the present disclosure, the second index sequence can be incorporated into the indexed nucleic acids using a polymerase chain reaction.

In a method of the present disclosure, each compartment contains a second index sequence that is unique relative to the index sequences in the other compartments. Alternatively, no more than about 50% of the compartments, *e.g*., no more than about 45%, no more than about 40%, no more than about 35%, no more than about 30%, no more than about 25%, no more than about 20%, no more than about 15%, no more than about 10%, no more than about 9%, no more than about 8%, no more than about 7%, no more than about 6%, no more than about 5%, no more than about 4%, no more than about 3%, no more than about 2% or no more than about 1% of the compartments, contain an second index sequence having the same nucleic acid sequence as the second index sequences of other compartments. In a method of the present disclosure, no more than 10% of the compartments contain a second index sequence having the same nucleic acid sequence as the second index sequences of other compartments. In a method of the present disclosure, no more than 5% of the compartments contain a second index sequence having the same nucleic acid sequence as the second index sequences of other compartments.

In a method of the present disclosure, the second index sequence is provided in a second polynucleotide. In a method of the present disclosure, the second polynucleotide further comprises a sequence that is reverse-complementary to a sequence of an indexed nucleic acid. In a method of the present disclosure, the second polynucleotide further comprises a sequence that is reverse-complementary to a sequence of the first polynucleotide. In a method of the present disclosure, the reverse-complementarity enables amplification, by polymerase chain reaction or linear amplification or other amplification methods. In a method of the present disclosure, the second polynucleotide comprises a molecular handle that mediates its ligation with an indexed nucleic acid. In a method of the present disclosure, the second polynucleotide can comprise a sequence that can bind a primer, *e.g*., a primer binding site, or function as a primer. For example, but not by way of limitation, the second polynucleotide comprises a sequence that can bind a primer for use in sequencing the indexed nucleic acid and/or for the amplification process.

In a method of the present disclosure, each compartment contains reagents for incorporating the second index sequence into the indexed nucleic acids. For example, but not by way of limitation, the reagents can include reagents for performing an amplification or ligation process in the compartments. Non-limiting examples of such reagents are disclosed herein and include polymerases, nucleoside triphosphates or NTP analogues, primers, cofactors, ligation reaction reagents, endonucleases, dyes, markers and labels. In a method of the present disclosure, each compartment can further include lysis reagents.

The method can further comprise lysing the cells. For example, but not by way of limitation, the cells can be lysed using sodium hydroxide, potassium hydroxide, sodium dodecyl sulfate, a non-ionic surfactant, a saponin, a proteinase, a lytic enzyme, a freeze thaw process, ultraviolet light and/or heat. In a method of the present disclosure, the cells are lysed after the incorporation of the second index sequence into the indexed nucleic acids to generate dual-indexed nucleic acids. Alternatively, the cells are lysed within the compartments containing the second index sequence but prior to incorporation of the second index sequence into the indexed nucleic acids. In a method of the present disclosure, the cells are lysed after the second round of compartmentalization but prior to addition of reagents for incorporating the second index sequence.

The method can further comprise incorporating one or more additional index sequences into the dual-indexed nucleic acids. In a method of the present disclosure one or more additional index sequences, *e.g*., two or more, three or more or four or more additional index sequences, can be incorporated into the dual-indexed nucleic acids. For example, but not by way of limitation, the methods of the present disclosure can further comprise incorporating a third index sequence into the dual-indexed nucleic acids to generate triple-indexed nucleic acids. In a method of the present disclosure, incorporating a third index sequence into the dual-indexed nucleic acids can be performed in another plurality of compartments containing a third index sequence as described about for the second index sequence. The methods of the present disclosure can further comprise incorporating a fourth index sequence into the triple-indexed nucleic acids to generate quadruple-indexed nucleic acids. The incorporation of one or more additional index sequences into the dual-indexed nucleic acids can include providing an additional index sequence, *e.g*., a third index sequence, that can couple to the first and/or second index sequence to generate triple-indexed nucleic acids. In a method of the present disclosure, the additional index sequence, *e.g*., the third index sequence, can be ligated to the first and/or second index sequence to generate triple-indexed nucleic acids. In a method of the present disclosure, a fourth index sequence can be coupled to the first, second and/or third index sequence, *e.g*., the fourth index sequence can be ligated to the third index sequence to generate quadruple-indexed nucleic acids.

A method of the present disclosure can further include performing a cell hashing technique. For example, but not by way of limitation, a method of the present disclosure can further include contacting the cells and/or nuclei with a reagent that binds to a target protein (referred to herein as a "protein binding reagent"). The protein binding reagent can be a reagent that specifically binds to a target protein, *e.g.,* specifically binds to a target protein of a cell and/or nucleus in a sample. Non-limiting examples of protein binding reagents include antibodies or antibody binding fragments thereof, aptamers, peptides and small molecules. the protein binding reagent can be an antibody or an antibody binding fragment thereof.

In a method of the present disclosure, a protein that is bound by a protein binding reagent can be any protein that is present in or on the surface of a cell. For example, but not by way of limitation, the target protein can be an intracellular protein, an extracellular protein or a transmembrane protein. In a method of the present disclosure, the target protein is a mutated form of a protein or a wild type form of a protein. In a method of the present disclosure, the target protein is an exogenously expressed protein. In a method of the present disclosure, the target protein is an endogenous protein. In a method of the present disclosure, the protein is a lineage specific protein. In a method of the present disclosure, the protein is cell specific protein (*e.g*., allow for distinguishing between two or more cell types).

In a method of the present disclosure, the protein binding reagent can be coupled to an oligonucleotide. In a method of the present disclosure, the oligonucleotide conjugated to the protein binding reagent can include a barcode (referred to herein as "hashtag"). In a method of the present disclosure, the protein binding reagent can be an antibody (or fragment thereof) coupled to an oligonucleotide comprising a barcode. In a method of the present disclosure, the barcode allows for identification of the protein binding reagent, *e.g*., antibody, and the corresponding protein to which it binds. In a method of the present disclosure, the protein binding reagent can be a TotalSeq^{™} antibody (BioLegend, San Diego, CA). In a method of the present disclosure, the use of a protein binding reagent, e.g., antibody, allows for the detection of a protein in a single sample (*e.g*., in a single cell in the sample). In a method of the present disclosure, the use of a protein binding reagent, e.g., antibody, allows for the quantification of a protein in a single sample (*e.g*., in a single cell in the sample).

In a method of the present disclosure, a method of the present disclosure can include contacting the cells and/or nuclei with a plurality of protein binding reagents. For example, but not by way of limitation, each protein binding reagent of the plurality of protein binding reagents specifically binds to a single target protein. In a method of the present disclosure, each protein binding reagent is coupled to a unique barcode. In a method of the present disclosure, a plurality of protein binding reagents can be used for binding a plurality of different target proteins in a sample. In a method of the present disclosure, the use of a plurality of protein binding reagents, *e.g*., antibodies, allows for the detection of multiple proteins in a single sample (*e.g*., in a single cell in the sample). In a method of the present disclosure, the use of multiple protein binding reagents, *e.g*., antibodies, allows for the quantification of multiple proteins in a single sample (*e.g*., in a single cell in the sample).

In a method of the present disclosure, the cells and/or nuclei can be contacted with the protein binding reagent prior to contacting the cells and/or nucleic with the plurality of particles (*e.g*., to perform a combinatorial indexing method of the present disclosure). In a method of the present disclosure, the cells and/or nuclei can be contacted with protein binding reagents prior to fixation (*e.g*., methanol fixation) of the cells and/or nucleic with the plurality of particles. In a method of the present disclosure, a subset of the cells and/or nuclei that are to be contacted with the plurality of particles can be contacted with a protein binding reagent (*e.g*., a protein binding reagent coupled to an oligonucleotide comprising a barcode) and a different subset of cells and/or nuclei that are to be contacted with the plurality of particles can be contacted with a different protein binding reagent (*e.g*., a different protein binding reagent coupled to an oligonucleotide comprising a different barcode). Alternatively or additionally, the cells and/or nuclei that are to be contacted with the plurality of particles can be contacted with a plurality of protein binding reagents. In a method of the present disclosure, at least two or more protein binding reagents, at least three or more protein binding reagents, at least four or more protein binding reagents, at least five or more protein binding reagents, at least six or more protein binding reagents, at least seven or more protein binding reagents, at least eight or more protein binding reagents, at least nine or more protein binding reagents, at least ten or more protein binding reagents can be used in the present disclosure, at least 50 or more protein binding reagents, at least 100 or more protein binding reagents, at least 150 or more protein binding reagents, at least 200 or more protein binding reagents, at least 250 or more protein binding reagents or at least 300 or more protein binding reagents, where each protein binding reagent specifically binds to a single target protein and each protein binding reagent is coupled to a unique barcode. In a method of the present disclosure, the sample (*e.g*., the plurality of cells and/or nuclei) can be contacted with the protein binding reagent, *e.g.,* the antibody specific for the target protein coupled to a barcode, for amount of time and under conditions to support specific binding of the protein binding reagent to the target protein.

A method of the present disclosure can further include treating the cells and/or nuclei with an agent, *e.g.,* a therapeutic agent. For example, but not by way of limitation, a method of the present disclosure can include contacting the cells and/or nuclei with an agent (*e.g*., a therapeutic agent), *e.g*., prior to performing a combinatorial indexing method described herein. A method of the present disclosure can include contacting the cells and/or nuclei with an agent, *e.g.,* a therapeutic agent, for at least about 15 minutes, at least about 1 hour, at least about 2 hours, at least about 6 hours, at least about 10 hours, at least about 12 hours, at least about 18 hours, at least about 24 hours or at least about 48 hours prior to performing a combinatorial indexing method described herein. A method of the present disclosure can include contacting the cells and/or nuclei with an agent, *e.g.,* a therapeutic agent, from about 15 minutes to about 48 hours (*e.g*., from about 15 minutes to about 24 hours, from about 15 minutes to about 18 hours, from about 15 minutes to about 12 hours, from about 15 minutes to about 10 hours, from about 15 minutes to about 6 hours, from about 15 minutes to about 2 hours, from about 30 minutes to about 48 hours, from about 1 hour to about 48 hours, from about 2 hours to about 48 hours, from about 6 hours to about 48 hours, from about 10 hours to about 48 hours, from about 12 hours to about 48 hours, from about 18 hours to about 48 hours, from about 24 hours to about 48 hours, from about 1 hour to about 48 hours, from about 2 hours to about 24 hours or from about 2 hours to about 12 hours. In a method of the present disclosure, the therapeutic agent is from a library of therapeutic agents and the methods of the present disclosure can be used to identify a therapeutic agent that would have a therapeutic effect. Non-limiting examples of therapeutic agents are described herein and can include polypeptide therapeutics, *e*.*g*., antibody-based therapeutics, oligonucleotides, cell-based therapeutics, gene editing systems and small molecule therapeutics.

A method of the present disclosure can further include subjecting the cells and/or nuclei to gene modulation or regulation. A method of the present disclosure can further include subjecting the cells and/or nuclei to a genome screen, *e.g*., prior to performing a combinatorial indexing method described herein. For example, and not by way of limitation, a method of the present disclosure can further include contacting the contacting the cells and/or nuclei with a gene editing system, *e.g*., prior to performing a combinatorial indexing method. Non-limiting examples of gene editing systems include homing endonucleases or meganucleases, ZFN, TALENs and CRISPR gene editing systems. In a method of the present disclosure, a method of the present disclosure can further include subjecting the cells and/or nuclei to a CRISPR-based screen, *e.g*., prior to performing a combinatorial indexing method described herein.

An exemplary method for combinatorial indexing nucleic acids of single cells or nuclei can comprise: (a) contacting a plurality of cells or nuclei isolated from a plurality of cells with a plurality of particles, *e.g*., microbeads, comprising a first index sequence; (b) partitioning individual particles and one or more of the cells or nuclei into an emulsion, *e.g*., emulsion droplets; and (c) incorporating the first index sequence into nucleic acids of the cells or nuclei to generate indexed nucleic acids; (d) combining the cells or nuclei that contain or associate with the indexed nucleic acids from a plurality of the emulsions, e.g., emulsion droplets, to generate pooled cells or nuclei; (e) distributing subsets of the pooled cells or nuclei into a plurality of compartments comprising a second index sequence; and (f) incorporating the second index sequence into the indexed nucleic acids in each compartment to generate dual-indexed nucleic acids. In a method of the present disclosure, the method can include (a) contacting a plurality of cells or nuclei isolated from a plurality of cells with a plurality of protein binding reagents, *e.g.*, a plurality of antibodies, coupled to an oligonucleotide comprising a barcode; (b) contacting the plurality of cells or nuclei isolated from the plurality of cells with a plurality of particles, *e.g*., microbeads, comprising a first index sequence; (c) partitioning individual particles and one or more of the cells or nuclei into an emulsion, *e.g*., emulsion droplets; and (d) incorporating the first index sequence into nucleic acids of the cells or nuclei to generate indexed nucleic acids; (e) combining the cells or nuclei that contain or associate with the indexed nucleic acids from a plurality of the emulsions, *e.g*., emulsion droplets, to generate pooled cells or nuclei; (f) distributing subsets of the pooled cells or nuclei into a plurality of compartments comprising a second index sequence; and (g) incorporating the second index sequence into the indexed nucleic acids in each compartment to generate dual-indexed nucleic acids. The method can further comprise incorporating a third index sequence into the dual-indexed nucleic acids to generate triple-indexed nucleic acids. In a method of the present disclosure, the plurality of cells or nuclei have been subjected to a genome screen, subjected to a cell hashing technique, subjected to a lineage tracing method and/or treated with a therapeutic agent prior to incorporating a first index sequence into the nucleic acids of the plurality of cells or nuclei. In a method of the present disclosure, the plurality of cells or nuclei can be subjected to a genome screen, subjected to a cell hashing technique, subjected to a lineage tracing method and/or treated with a therapeutic agent prior to contacting the cells or nuclei to the protein binding reagents.

An exemplary method for combinatorial indexing nucleic acids of single cells or nuclei can comprise: (a) providing pooled cells or nuclei associated with or comprising nucleic acids comprising a first index sequence, wherein the first index sequence is incorporated into the nucleic acids of the pooled cells or nuclei in emulsion droplets; (b) distributing subsets of the pooled cells or nuclei into a plurality of compartments comprising a second index sequence; and (c) incorporating the second index sequence into the nucleic acids in each compartment to generate dual-indexed nucleic acids. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets; (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. In a method of the present disclosure, the pooled cells or nuclei have been subjected to a genome screen, subjected to a cell hashing technique, subjected to a lineage tracing method and/or treated with a therapeutic agent prior to incorporating a first index sequence into the nucleic acids of the pooled cells or nuclei.

The method can include: (a) providing pooled cells or nuclei associated with or comprising nucleic acids comprising a first index sequence, wherein the first index sequence is incorporated into the nucleic acids of the pooled cells or nuclei in emulsion droplets, wherein the pooled cells or nuclei are bound by protein binding reagents, *e.g.*, antibodies, coupled to an oligonucleotide comprising a barcode; (b) distributing subsets of the pooled cells or nuclei into a plurality of compartments comprising a second index sequence; and (c) incorporating the second index sequence into the nucleic acids in each compartment to generate dual-indexed nucleic acids. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets: (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. In a method of the present disclosure, the pooled cells or nuclei can be subjected to a genome screen, subjected to a cell hashing technique, subjected to a lineage tracing method and/or treated with a therapeutic agent prior to incorporating a first index sequence into the nucleic acids of the pooled cells or nuclei, *e.g*., prior to step (a).

The present disclosure further provides methods for generating a sequencing library. The sequencing library comprises nucleic acids indexed by the methods of the present disclosure. For example, but not by way of limitation, a sequencing library in accordance with the instant disclosure comprises nucleic acids from single cells that are at least dual-indexed. In a method of the present disclosure, the nucleic acids comprising the sequencing library are triple-indexed. In a method of the present disclosure, the sequencing library is partially or fully representative of the transcriptome of one or more single cells, *e.g*., where all or a subset of the nucleic acids of a particular cell have been labeled with index sequences, *e.g*., a unique combination of index sequences. In a method of the present disclosure, the sequencing library partially or fully represents the genome of one or more single cells, *e.g.,* where all or a subset of the nucleic acids of a particular cell have been labeled with index sequences, *e.g*., a unique combination of index sequences.

In a method of the present disclosure, a method for generating a sequencing library can comprise combining multi-indexed nucleic acids, which can be prepared as disclosed herein, to generate a library from the plurality of single cells or nuclei. the multi-indexed nucleic acids, e.g., the dual-indexed nucleic acids, derived from a subset of the total cells or nuclei, generated using the methods of the present disclosure can be combined to prepare a sequencing library. For example, but not by way of limitation, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% of the cells' or nuclei' dual-indexed nucleic acids are combined to generate a sequencing library. In a method of the present disclosure, at least about 20% of the cells' or nuclei' dual-indexed nucleic acids are combined to generate a sequencing library. In a method of the present disclosure, at least about 50% of the cells' or nuclei' dual-indexed nucleic acids are combined to generate a sequencing library. In a method of the present disclosure, at least about 90% of the cells' or nuclei' dual-indexed nucleic acids are combined to generate a sequencing library. In a method of the present disclosure, all of the multi-indexed nucleic acids, *e.g*., the dual-indexed nucleic acids, can be combined to generate a sequencing library.

In a method of the present disclosure, less than all of the multi-indexed nucleic acids, *e.g.,* the dual-indexed nucleic acids, can be combined to generate a sequencing library. For example, but not by way of limitation, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, less than about 90%, less than about 91%, less than about 92%, less than about 93%, less than about 94%, less than about 95%, less than about 96%, less than about 97%, less than about 98% or less than about 99% of the cells' or nuclei' dual-indexed nucleic acids are combined to generate a sequencing library. In a method of the present disclosure, less than about 50% of the multi-indexed nucleic acids, *e.g.,* the dual-indexed nucleic acids, can be combined to generate a sequencing library. In a method of the present disclosure, less than about 90% of the multi-indexed nucleic acids, *e.g*., the dual-indexed nucleic acids, can be combined to generate a sequencing library.

In a method of the present disclosure, from about 10% to about 99% of the cells' or nuclei' dual-indexed nucleic acids are combined to generate a sequencing library. In a method of the present disclosure, from about 10% to about 95%, from about 10% to about 90%, from about 10% to about 80%, from about 10% to about 70%, from about 10% to about 60%, from about 10% to about 50%, from about 10% to about 40%, from about 10% to about 30%, from about 10% to about 20%, from about 20% to about 99%, from about 30% to about 99%, from about 40% to about 99%, from about 50% to about 99%, from about 60% to about 99%, from about 70% to about 99%, from about 80% to about 99%, from about 90% to about 99%, from about 20% to about 80% or from about 30% to about 50% of the cells' or nuclei' dual-indexed nucleic acids are combined to generate a sequencing library.

In a method of the present disclosure, a method for generating a sequencing library comprising nucleic acids from a plurality of single cells can comprise: (a) contacting a plurality of cells or nuclei isolated from a plurality of cells with a plurality of particles, *e.g*., microbeads, comprising a first index sequence; (b) partitioning individual particles, *e.g*., microbeads, and one or more of the cells or nuclei into emulsion droplets; (c) incorporating the first index sequence into nucleic acids of the cells or nuclei to generate indexed nucleic acids; (d) combining the cells or nuclei that contain or associate with the indexed nucleic acids from a plurality of the emulsion droplets to generate pooled indexed cells or nuclei; (e) distributing subsets of the pooled cells or nuclei into a plurality of compartments comprising a second index sequence; (f) incorporating the second index sequence into the indexed nucleic acids in each compartment to generate dual-indexed nucleic acids; and (g) combining all or a subset of the dual-indexed nucleic acids to generate a sequencing library from the plurality of cells or nuclei. In a method of the present disclosure, the plurality of cells or nuclei have been subjected to a genome screen, subjected to a cell hashing technique, subjected to a lineage tracing method and/or treated with a therapeutic agent prior to incorporating a first index sequence into the nucleic acids of the plurality of cells or nuclei, *e.g*., prior to step (a).

A method of the present disclosure can include a cell hashing technique. For example, but not by way of limitation, a method of the present disclosure includes: (a) contacting a plurality of cells or nuclei isolated from a plurality of cells with a plurality of protein binding reagents, *e.g*., a plurality of antibodies, coupled to an oligonucleotide comprising a barcode; (b) contacting the plurality of cells or nuclei isolated from the plurality of cells with a plurality of particles, *e.g*., microbeads, comprising a first index sequence; (c) partitioning individual particles, *e.g*., microbeads, and one or more of the cells or nuclei into emulsion droplets; (d) incorporating the first index sequence into nucleic acids of the cells or nuclei to generate indexed nucleic acids; (e) combining the cells or nuclei that contain or associate with the indexed nucleic acids from a plurality of the emulsion droplets to generate pooled indexed cells or nuclei; (f) distributing subsets of the pooled cells or nuclei into a plurality of compartments comprising a second index sequence; (g) incorporating the second index sequence into the indexed nucleic acids in each compartment to generate dual-indexed nucleic acids; and (h) combining all or a subset of the dual-indexed nucleic acids to generate a sequencing library from the plurality of cells or nuclei. The plurality of cells or nuclei can be further subjected to a genome screen, subjected to a lineage tracing method and/or treated with a therapeutic agent, prior to incorporating a first index sequence into the nucleic acids of the plurality of cells or nuclei and/or prior to contacting the plurality of cells or nuclei with the plurality of protein binding reagents, *e.g*., prior to step (a).

A method for generating a sequencing library comprising nucleic acids from a plurality of single cells comprises: (a) providing pooled cells or nuclei associated with or comprising nucleic acids comprising a first index sequence, wherein the first index sequence is incorporated into the nucleic acids of the pooled cells or nuclei in emulsion droplets; (b) distributing subsets of the pooled cells or nuclei into a plurality of compartments comprising a second index sequence; (c) incorporating the second index sequence into the nucleic acids in each compartment to generate dual-indexed nucleic acids; and (d) combining all or a subset of the dual-indexed nucleic acids to generate a sequencing library from the plurality of cells or nuclei. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets; (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. The pooled cells or nuclei can be subjected to a genome screen, subjected to a cell hashing technique, subjected to a lineage tracing method and/or treated with a therapeutic agent, prior to incorporating a first index sequence into the nucleic acids of the pooled cells or nuclei.

A method of the present disclosure, includes: (a) providing pooled cells or nuclei associated with or comprising nucleic acids comprising a first index sequence, wherein the first index sequence is incorporated into the nucleic acids of the pooled cells or nuclei in emulsion droplets, wherein the pooled cells or nuclei are bound by protein binding reagents, e.g., antibodies, coupled to an oligonucleotide comprising a barcode; (b) distributing subsets of the pooled cells or nuclei into a plurality of compartments comprising a second index sequence; (c) incorporating the second index sequence into the nucleic acids in each compartment to generate dual-indexed nucleic acids; and (d) combining all or a subset of the dual-indexed nucleic acids to generate a sequencing library from the plurality of cells or nuclei. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets; (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. In a method of the present disclosure, the pooled cells or nuclei have been further subjected to a genome screen, subjected to a lineage tracing method and/or treated with a therapeutic agent, prior to incorporating a first index sequence into the nucleic acids of the pooled cells or nuclei.

In another aspect, the present disclosure further provides methods for sequencing a library generated according to the methods disclosed herein. In a method of the present disclosure, the sequencing methods of the present disclosure comprise sequencing multi-indexed nucleic acids, *e.g*., dual-indexed nucleic acids, from single cells. In a method of the present disclosure, the sequencing methods of the present disclosure comprise sequencing of dual-indexed nucleic acids that are partially or fully representative of the transcriptome of single cells. The sequencing methods of the present disclosure can comprise the sequencing of dual-indexed nucleic acids that are partially or fully representative of the genome of single cells.

In a method of the present disclosure, a sequencing method of the present disclosure comprises sequencing all or a subset of the multi-indexed nucleic acids, *e.g*., the dual-indexed nucleic acids, of a library. For example, but not by way of limitation, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% of the multi-indexed nucleic acids, *e.g.,* the dual-indexed nucleic acids, included in the library can be sequenced. In a method of the present disclosure, from about 10% to about 99% of the dual-indexed nucleic acids can be sequenced. In a method of the present disclosure, from about 10% to about 95%, from about 10% to about 90%, from about 10% to about 80%, from about 10% to about 70%, from about 10% to about 60%, from about 10% to about 50%, from about 10% to about 40%, from about 10% to about 30%, from about 10% to about 20%, from about 20% to about 99%, from about 30% to about 99%, from about 40% to about 99%, from about 50% to about 99%, from about 60% to about 99%, from about 70% to about 99%, from about 80% to about 99%, from about 90% to about 99%, from about 20% to about 80% or from about 30% to about 50% dual-indexed nucleic acids can be sequenced.

The present disclosure provides a method for sequencing the nucleic acids from a plurality of cells that comprises: (a) contacting a plurality of cells or nuclei isolated from a plurality of cells with a plurality of particles, *e.g*., microbeads, comprising a first index sequence; (b) partitioning individual particles, *e.g*., microbeads, and one or more of the cells or nuclei into emulsion droplets; (c) incorporating the first index sequence into nucleic acids of the cells or nuclei to generate indexed nucleic acids; (d) combining the cells or nuclei that contain or associate with the indexed nucleic acids from a plurality of the emulsion droplets to generate pooled indexed cells or nuclei; (e) distributing subsets of the pooled cells or nuclei into a plurality of compartments comprising a second index sequence; (f) incorporating the second index sequence into the indexed nucleic acids in each compartment to generate dual-indexed nucleic acids; (g) combining all or a subset of the dual-indexed nucleic acids to generate a sequencing library from the plurality of cells; and (h) sequencing all or a subset of the dual-index nucleic acids. In a method of the present disclosure, the plurality of cells or nuclei have been subjected to a genome screen, subjected to a cell hashing technique, subjected to a lineage tracing method and/or treated with a therapeutic agent prior to incorporating a first index sequence into the nucleic acids of the plurality of cells or nuclei.

In a method of the present disclosure, the method comprises: (a) contacting a plurality of cells or nuclei isolated from a plurality of cells with a plurality of protein binding reagents, *e.g*., a plurality of antibodies, coupled to an oligonucleotide comprising a barcode; (b) contacting the plurality of cells or nuclei isolated from the plurality of cells with a plurality of particles, *e.g*., microbeads, comprising a first index sequence; (c) partitioning individual particles, *e.g*., microbeads, and one or more of the cells or nuclei into emulsion droplets; (d) incorporating the first index sequence into nucleic acids of the cells or nuclei to generate indexed nucleic acids; (e) combining the cells or nuclei that contain or associate with the indexed nucleic acids from a plurality of the emulsion droplets to generate pooled indexed cells or nuclei; (f) distributing subsets of the pooled cells or nuclei into a plurality of compartments comprising a second index sequence; (g) incorporating the second index sequence into the indexed nucleic acids in each compartment to generate dual-indexed nucleic acids; (h) combining all or a subset of the dual-indexed nucleic acids to generate a sequencing library from the plurality of cells; and (i) sequencing all or a subset of the dual-index nucleic acids and/or sequencing the barcodes derived from the protein binding reagents. In a method of the present disclosure, the plurality of cells or nuclei have been further subjected to a genome screen, subjected to a lineage tracing method and/or treated with a therapeutic agent prior to incorporating a first index sequence into the nucleic acids of the plurality of cells or nuclei and/or prior to contacting the plurality of cells or nuclei with the plurality of protein binding reagents.

The present disclosure provides a method for sequencing the nucleic acids from a plurality of cells that comprises: (a) providing pooled cells or nuclei associated with or comprising nucleic acids comprising a first index sequence, wherein the first index sequence is incorporated into the nucleic acids of the pooled cells or nuclei in emulsion droplets; (b) distributing subsets of the pooled cells or nuclei into a plurality of compartments comprising a second index sequence; (c) incorporating the second index sequence into the nucleic acids in each compartment to generate dual-indexed nucleic acids; (d) combining all or a subset of the dual-indexed nucleic acids to generate a sequencing library from the plurality of cells; and (d) sequencing all or a subset of the dual-index nucleic acids. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets; (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. In a method of the present disclosure, the pooled cells or nuclei have been subjected to a genome screen, subjected to a cell hashing technique, subjected to a lineage tracing method and/or treated with a therapeutic agent, prior to incorporating a first index sequence into the nucleic acids of the pooled cells or nuclei.

In a method of the present disclosure, the method comprises: (a) providing pooled cells or nuclei associated with or comprising nucleic acids comprising a first index sequence, wherein the first index sequence is incorporated into the nucleic acids of the pooled cells or nuclei in emulsion droplets, wherein the pooled cells or nuclei are bound by protein binding reagents, *e.g*., antibodies, coupled to an oligonucleotide comprising a barcode; (b) distributing subsets of the pooled cells or nuclei into a plurality of compartments comprising a second index sequence; (c) incorporating the second index sequence into the nucleic acids in each compartment to generate dual-indexed nucleic acids; (d) combining all or a subset of the dual-indexed nucleic acids to generate a sequencing library from the plurality of cells; and (d) sequencing all or a subset of the dual-index nucleic acids and/or sequencing the barcodes derived from the protein binding reagents. In a method of the present disclosure, the first index sequence is incorporated into the nucleic acids of the cells or nuclei in emulsion droplets by a method comprising: (i) contacting a plurality of cells or nuclei with a plurality of particles comprising the first index sequence; (ii) partitioning individual particles and one or more of the cells or nuclei into emulsion droplets; (iii) incorporating the first index sequence into nucleic acids of the cells or nuclei to generate indexed nucleic acids; and (iv) combining the cells or nuclei that associate with or comprise the indexed nucleic acids from a plurality of the emulsion droplets to generate the pooled cells or nuclei associated with or comprising nucleic acids comprising the first index sequence. In a method of the present disclosure, the pooled cells or nuclei have been further subjected to a genome screen, subjected to a lineage tracing method and/or treated with a therapeutic agent, prior to incorporating a first index sequence into the nucleic acids of the pooled cells or nuclei and/or prior to binding the protein binding reagents to the pooled cells or nuclei.

Appropriate sequencing methods can be used for sequencing the indexed nucleic acids. The nucleic acids can be sequenced by a high throughput sequencing method. The nucleic acids can be sequenced by next-generation sequencing (NGS). For example, but not by way of limitation, sequencing can be performed using the Illumina NGS platform. The nucleic acids can be sequenced by a pyrosequencing method. The nucleic acids can be sequenced by Sanger sequencing. The nucleic acids can be sequenced using nanopore-based sequencing. The sequence reads obtained using the methods of the present disclosure can be attributed to specific cells based upon the unique combination of the two indexed sequences.

### EXAMPLES

The presently disclosed subject matter will be better understood by reference to the following Examples, which are provided as exemplary of the presently disclosed subject matter, and not by way of limitation.

### Example 1: Combinatorial Indexing for OAK-scRNAseq

This example describes a combinatorial indexing method for sequencing the RNAs of single cells as shown in FIG. 1. In particular, this example describes the addition of two index sequences to the RNAs of single cells.

### Cell preparation:

The combinatorial indexing method begins by preparing single cell suspensions for fixation. If adherent cell lines or solid tissues were used, cell dissociation was performed prior to fixation. The cells were collected in a medium, and then washed with phosphate buffered saline (PBS). The washed cells were gently resuspended with 200 µl chilled PBS per 1x10⁶ cells to achieve single-cell suspensions.

The single cells were subsequently fixed by adding 800 µl of chilled pure methanol per 1x10⁶ cells drop by drop with gentle stirring by pipette tips to prevent cells from clumping. The samples are then stored at -20°C for 40 min. The fixed samples were placed on ice for 5 minutes and subsequently centrifuged at 1000g at 4°C for 5 min. The supernatant was removed and the cells were resuspended in a buffer include 3X SSC, 1% BSA, 1mM DTT and 0.2U/ul RNase inhibitor. The volume was adjusted with a buffer to achieve a concentration of 50,000 cells/µl.

### Integration of Primary Barcodes:

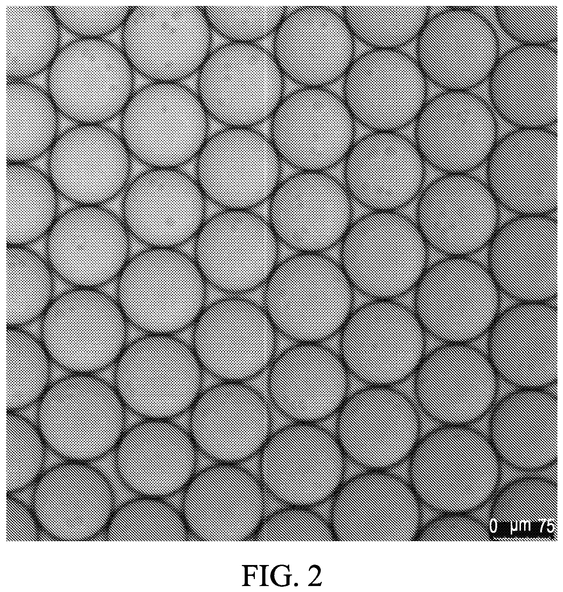

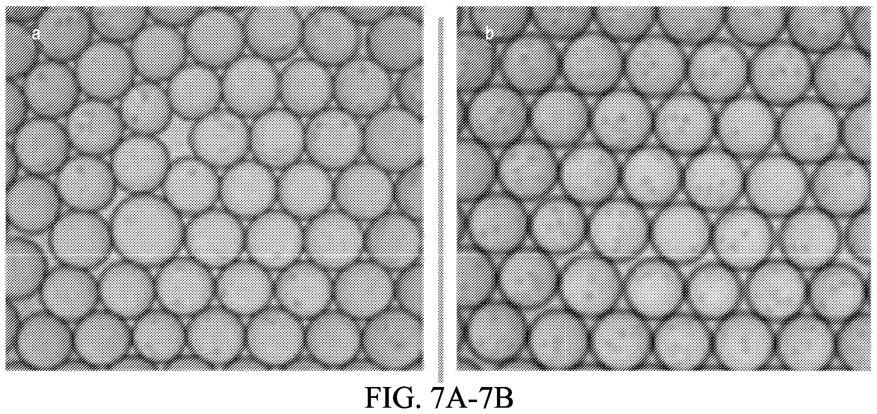

The Master Mix was prepared on ice based on the user guide for Chromium Next GEM Single Cell 3' Reagent Kits v3.1 (referred to herein as the "Chromium Next GEM Single Cell user guide"). 31.8 µl of the Master Mix for each sample to load was aliquoted. The volume of fixed cells to load for each sample using the following formula: Volume_CellStock (µl)=TargetedCellNumber * 2.3 / Concentration_CellStock. For example, if the cell stock concentration is 50,000 cells/µl to target 100,000 cells, 230,000 cells were loaded by using 100k*2.3/50k=4.6 µl cell stock as the input. For each channel, 43.3 µl of nuclease-free water was added to a tube that contains 31.8 µl of Master Mix based on the calculation in the previous step. Second, the corresponding volume of fixed cells was added. The cell-reagent mixture was mixed gently with a pipette, and the same pipette tip was used to dispense 70 µl of the mixture into a well in row labeled 1 on the chip. The previous step was repeated for each of the samples to load. Each sample was placed in a well. The rest of the wells were loaded according to the Chromium Next GEM Single Cell user guide. The GEM Gasket was attached and the Chromium Controller was run based on the Chromium Next GEM Single Cell user guide. After the run was completed, the GEMs were transferred according to the Chromium Next GEM Single Cell user guide. As shown in FIG. 2, this process resulted in the successful generation of multi-cell GEMs. FIG. 7A shows the generation of multi-cell GEMs by loading 150,000 cells per channel (resulting in the recovery of 85,000 cells and the sequencing of about 2,000-4,000 genes per cell) and FIG. 7B shows the generation of multi-cell GEMs by loading 450,000 cells per channel (resulting in the recovery of 273,000 cells and the sequencing of about 600 genes per cell). Representative droplets containing between 0 and 8 cells are shown. The transferred GEMs were then incubated in a thermal cycler at 53°C for 45 minutes to enable primary barcoding during a reverse transcription process, followed by a 4°C hold for a short period of time if necessary. At the end of the incubation, the GEMs were unpacked as follows.

### GEM Unpacking:

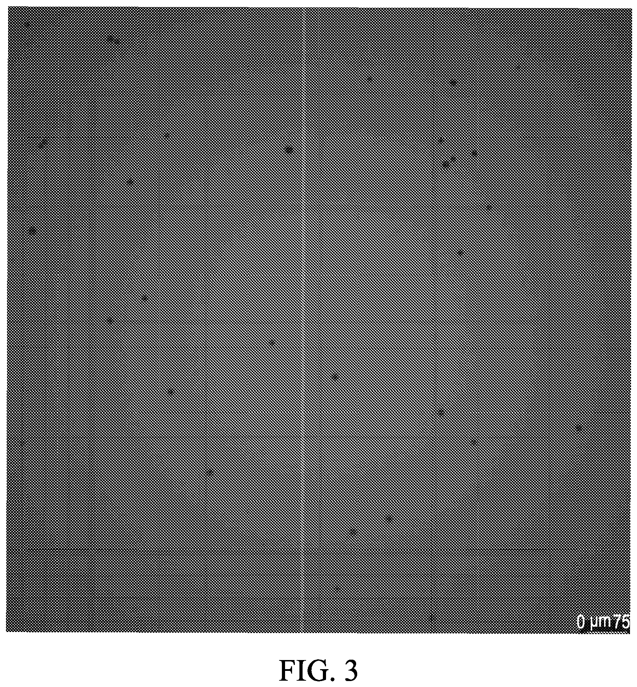

125 µl of Recovery Agent was added to each sample at room temperature, and after a 5-10 minute incubation a biphasic separation formed and stabilized. The aqueous phase was slowly transferred from the top of each sample tube to a round-bottom 2.0 mL Eppendorf tube. 500 µl of 3X SSC was added slowly to each sample. The tube was centrifuged in a swinging bucket centrifuge at 4°C for 650g for 5 min. The supernatant was removed without disrupting the cell pellets. 500 µl 3X SSC was slowly added to each sample without disrupting the cell pellets. The tube was centrifuged in a swinging bucket centrifuge at 4°C for 650g for 5 min, and the supernatant was removed without disrupting the cell pellets. The cells were resuspended in 3X SSC using a regular-bore P200 pipette tip. An appropriate resuspension volume should be used so that each 10 µl will contain 1,000 to 10,000 cells based on multiplet tolerance of each project. As shown in FIG. 3, cells were successfully recovered after the unpacking of the GEMs. Each sample was aliquoted into multiple individual PCR tubes so that each PCR tube contains 10 µl resuspended cells. Each 10 µl contains 1,000 to 10,000 cells. The aliquots were frozen at -80°C until next steps. In a thermal cycler, the aliquots were heated at 95°C for 5 minutes. 90 µl of EB was added to each aliquot, and cleanup was performed using Dynabeads MyOne Silane. The heating at 95°C and cleanup with Dynabeads MyOne Silane results in the lysis of the cells.

### Integration of Secondary Barcodes:

cDNA amplification was performed for each aliquot using a uniquely barcoded primer that hybridizes with the common sequence located upstream of primary cell barcodes and UMI in the first strand cDNA. The other primer in the PCR reaction covers the TSO side of the first strand cDNA, and therefore is shared across all aliquots. After the PCR reaction is complete, the products from different aliquots were pooled. The PCR products were cleaned up with 0.6X SPRIselect beads. 1 µl of the resulting cDNA was run in a D5000 tape to assess cDNA quantity and length distribution.

The cDNA library was then sequenced by using the Illumina sequencing library construction steps followed by sequencing. Table 1 shows the performance metrics for the OAK-scRNAseq method. As shown in Table 1, this method allows for the analysis of many cells with a low multiplet rate.

**Table 1**

| | |
|---|---|
| Median number of genes per cell (K562) | 1608 |
| Median number of genes per cell (NIH/3T3) | 628 |
| Multiplet rate | 4.4% |
| Fraction reads in cells | 80.7% |

### Example 2: Combinatorial Indexing for OAK-multiome (ATAC & RNA co-assay)

This example describes a combinatorial indexing method for sequencing the RNA and genomic DNA of single cells as shown in FIG. 1. In particular, this example describes the addition of two index sequences to the genomic DNA of single cells.

### Nuclei preparation:

The method begins by preparing single nuclei suspensions for fixation. The cells were collected in a medium. If adherent cell lines or solid tissues were used, cell dissociation can be performed prior to fixation. The cells were then washed with phosphate buffered saline (PBS), and gently resuspended with 200 µl chilled PBS per 1x10⁶ cells to achieve single-cell suspensions. Nuclei were subsequently isolated from the single-cell suspensions.

The single nuclei were subsequently fixed by adding the appropriate volumes of formaldehyde and PBS to the single-nuclei suspension so that 0.3% of formaldehyde was used for fixation. The samples were stored on ice for 15 minutes. The fixed nuclei were then washed with a buffer including PBS, 1% BSA and 0.2U/µl RNase Inhibitor. The fixed nuclei were resuspended gently with chilled diluted nuclei buffer by 10x Genomics. The volume was adjusted to achieve a desired concentration such as 4,000 nuclei/µl. Transposition for nuclei was performed according to Chromium Next GEM Single Cell Multiome ATAC + Gene Expression ("Chromium Next GEM Single Cell Multiome user guide"). Up to 20,000 nuclei were used in each of the transposition reaction of 15 µl reaction volume. In order to have enough samples for overloading of the microfluidic device, multiple transposition reactions were created in parallel. 10 of such reactions were created in parallel for each sample, so that a total of 200,000 nuclei were transposed for each sample. If additional ATAC enzyme is needed, TDE1 enzyme from the Illumina Tagment DNA TDE1 Enzyme and Buffer Kits can be used. Alternatively, homebrew Tn5 enzymes loaded with appropriate adapters can be used. After the transposition reaction, for each sample, the nuclei were collected from all the reaction tubes into a round-bottom 2.0 mL Eppendorf tube. The tubes were centrifuged in a swinging bucket centrifuge at 4°C for 500g for 5 min. Most of the supernatant was removed without disrupting the nucleus pellets. The last 15 µl supernatant was left in the tube for each sample. The transposed nuclei were gently resuspended with the remaining buffer.

### Integration of Primary Barcodes:

The Master Mix was prepared on ice based on the Chromium Next GEM Single Cell Multiome user guide. 60 µl of the Master Mix was added to a sample tube containing the 15 µl of transposed and concentrated nuclei.

The nuclei-reagent mixture was mixed gently with a pipette, and the same pipette tip was used to dispense 70 µl of the mixture into a well in row labeled 1 on the chip. The previous step was repeated for each of the samples to load. Each sample was placed in a well. The rest of the wells were loaded according to the Chromium Next GEM Single Cell Multiome user guide. The GEM Gasket was attached and the Chromium Controller was run based on the user guide. After the run was completed, the GEMs were transferred according to the Chromium Next GEM Single Cell Multiome user guide. 1 µl of the GEMs can be inspected under a microscope. The transferred GEMs were then incubated in a thermal cycler at 37°C for 45 min followed by 25°C at 30 minutes to enable primary barcoding, followed by a 4°C hold for a short period of time if necessary. At the end of the incubation, 5 µl of Quenching Agent was added to each sample according to the user guide, and the GEMs were unpacked as follows.

### GEM Unpacking:

125 µl of Recovery Agent was added to each sample at room temperature, and after a 5-10 minute incubation a biphasic separation formed and stabilized. The aqueous phase was slowly transferred from the top of each sample tube to a round-bottom 2.0 mL Eppendorf tube. 500 µl of 3X SSC was added slowly to each sample. The tube was centrifuged in a swinging bucket centrifuge at 4°C for 650g for 5 min. The supernatant was removed without disrupting the cell pellets. 500 µl 3X SSC was slowly added to each sample without disrupting the nuclei pellets. The tube was centrifuged in a swinging bucket centrifuge at 4°C for 650g for 5 min, and the supernatant was removed without disrupting the nuclei pellets. The nuclei were resuspended in 3X SSC using a regular-bore P200 pipette tip. An appropriate resuspension volume should be used so that each 10 µl will contain 1,000 to 10,000 cells based on multiplet tolerance of each project. Each sample was aliquoted into multiple individual PCR tubes so that each PCR tube contains 10 µl resuspended nuclei. Each 10 µl contains 1,000 to 10,000 nuclei. The aliquots were frozen at -80°C until next steps. In a thermal cycler, the aliquots were heated at 80°C for 10 minutes. 90 µl of EB was added to each aliquot, and cleanup was performed using Dynabeads MyOne Silane.

### Integration of Secondary Barcodes:

For each aliquot, a pre-amplification was performed for cDNA and ATAC fragments with a mixture of primers that amplify both cDNA and ATAC fragments. The PCR products for each aliquot were cleaned with 1.6X SPRI Select beads. 25% of the products of each aliquot was used from the previously step for cDNA amplification with a uniquely barcoded primer that hybridizes with the common sequence located upstream of primary cell barcodes and UMI in the first strand cDNA. The other primer in the PCR reaction covers the TSO side of the first strand cDNA, and therefore is shared across all aliquots. Products from different aliquots can be pooled after the PCR reaction is completed. The cDNA amplification products were cleaned up with 0.6X SPRIselect beads. 1 µl of the resulting cDNA was run in a D5000 tape to assess cDNA quantity and length distribution.

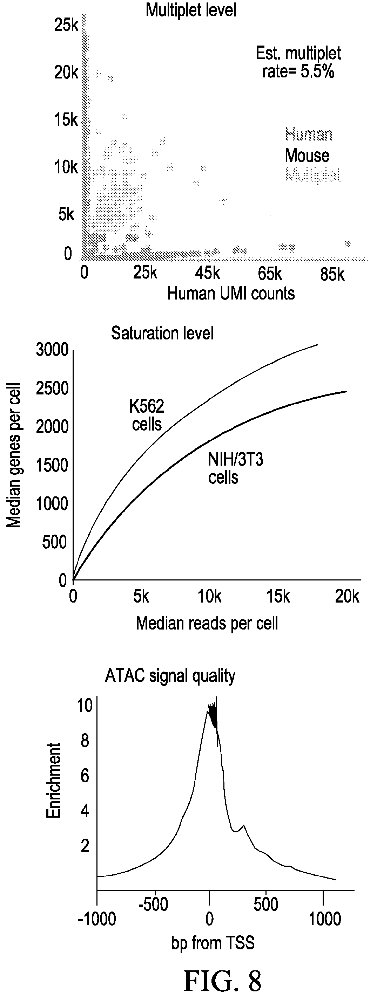

The cDNA library was then sequenced by using the Illumina sequencing library construction steps followed by sequencing. Table 2 and FIG. 8 shows the performance metrics for the OAK-multiome method. As shown in Table 2, this method allows for the analysis of many cells with a low multiplet rate.

**Table 2**

| | |
|---|---|
| Fraction reads in cells | 97.1% |
| Multiplet rate | 5.5% |
| Median number of genes per cell (K562) | 3348 |
| Median number of genes per cell (NIH/3T3) | 2607 |
| Enrichment score of TSS | 11.62 |
| Median fragments/cell (K-562) | 12821 |
| Median fragments/cell (NIH/3T3) | 10016 |

### Example 3: OAK Combinatorial Indexing with Cell Hashing

This example discloses a combinatorial indexing method for sequencing the RNAs of single cells that have been labeled with antibodies coupled to barcoded oligonucleotides (referred to herein as "hashtags").

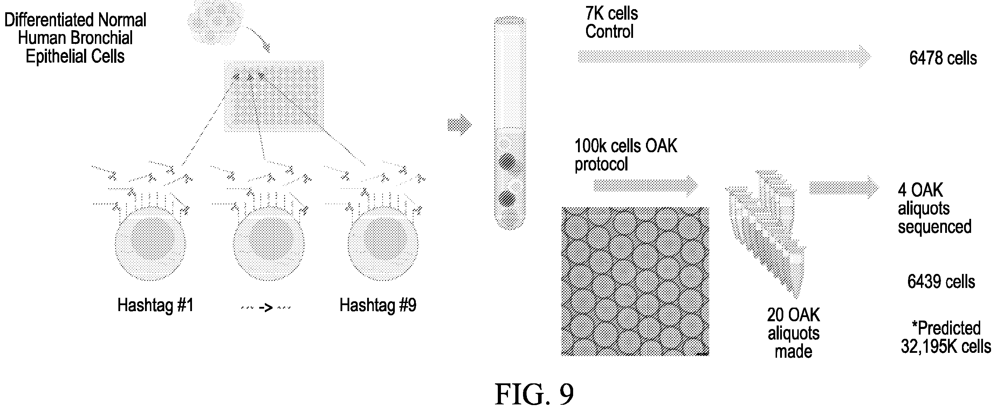

As shown in FIG. 9, differentiated human bronchial epithelial cells were hash-tagged using antibodies coupled to an oligonucleotide comprising a barcode (*i.e.,* hashtag) and further processed using a combinatorial indexing method or a control method. In particular, primary lung cells were grown in plates and stained with commercially available hashtag antibodies specific for beta2-microglobulin B2M and CD298, which is expressed on all cells, and coupled to oligonucleotides comprising known barcodes. A total of 9 different barcodes were used. After antibody binding, the cells were washed, then pooled and sorted by cytometry for viability. 100,000 cells were further processed using the OAK method described in Example 1 and 7,000 cells were used for droplet sequencing utilizing the 10x Chromium single cell 3' reagent kit and the Chromium platform as a control.

For the OAK method, the first index sequences were added to the cDNA and the antibody-derived oligonucleotide in the droplets. The antibody-derived oligonucleotide and the cDNA from the RNA were tagged with the same first index sequence in a droplet, thereby allowing the RNA and the antibody-derived sequences to be linked in the data. After unpacking of the emulsion, the cells were placed in different aliquots for the integration of the secondary barcodes. Integration of the secondary barcodes was performed as follows: for each aliquot, an amplification was performed for cDNA and antibody (protein or hashtag)-derived oligonucleotides with a mixture of primers that amplify both cDNA and antibody-derived oligonucleotides. During this reaction, a secondary index is added to the cDNA. The PCR products for each aliquot were separated with SPRI Select beads. 0.6X SPRI Select beads were used to collect the cDNA for gene expression libraries analogous to Example 1. The supernatant was kept from the initial separation from cDNA for further clean-up using 2.1X SPRI Select beads to obtain the antibody-derived oligonucleotides. Amplification of the antibody-derived oligonucleotides used a uniquely barcoded primer that hybridizes with the common sequence located upstream of primary cell barcodes and the other primer in the PCR reaction covers a common sequence in the antibody-derived oligonucleotide, and, therefore, shared across all aliquots.

Sequencing libraries from the transcriptome and the hashtags were then deplexed for analysis.

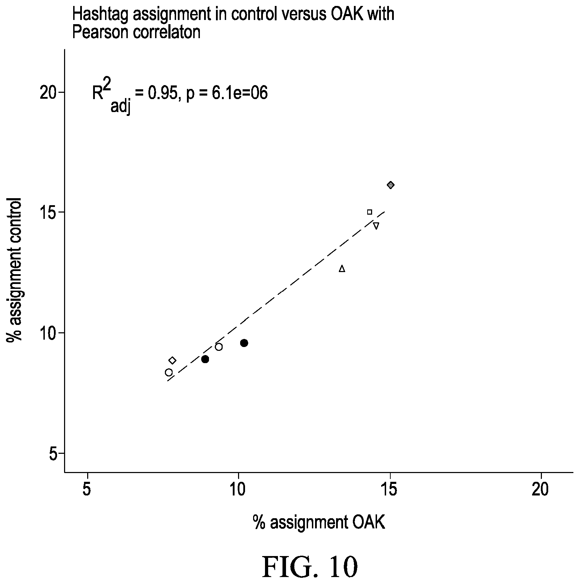

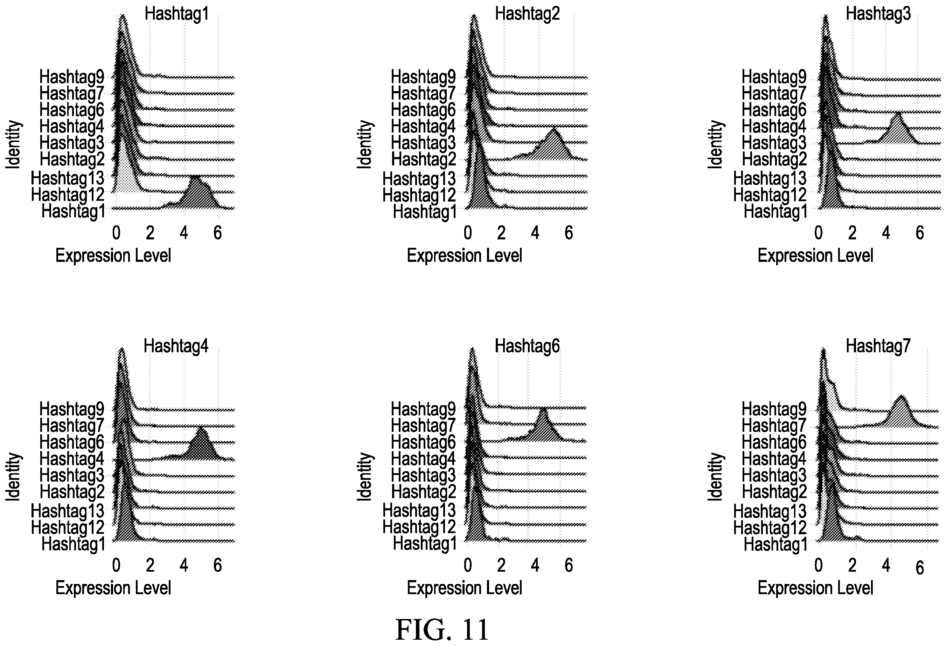

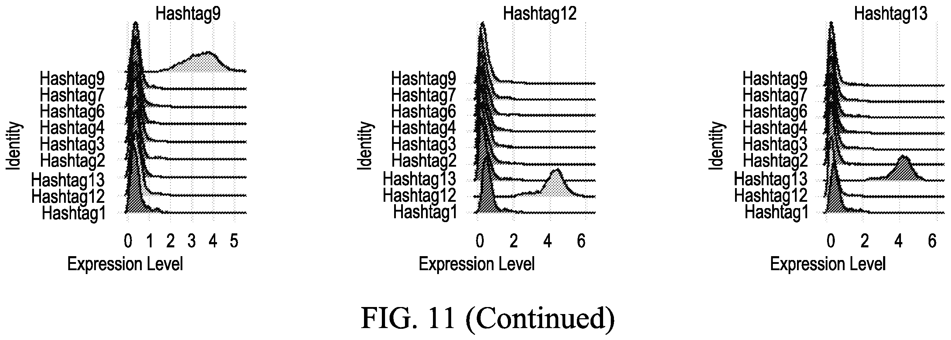

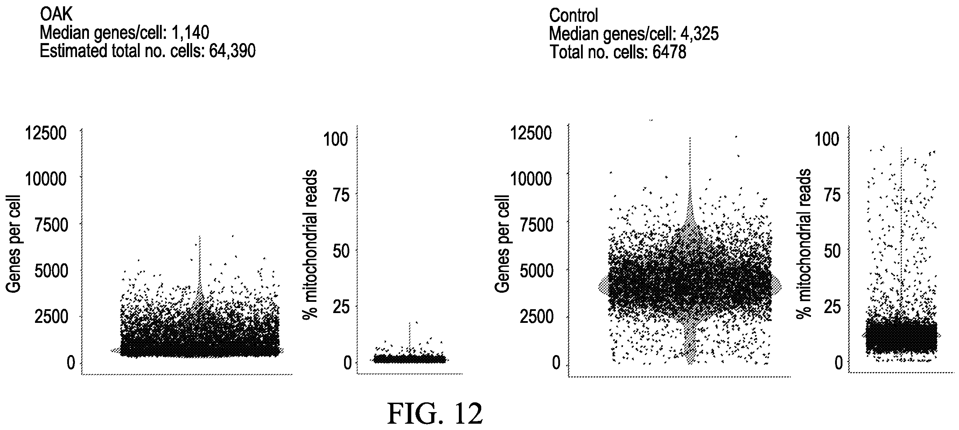

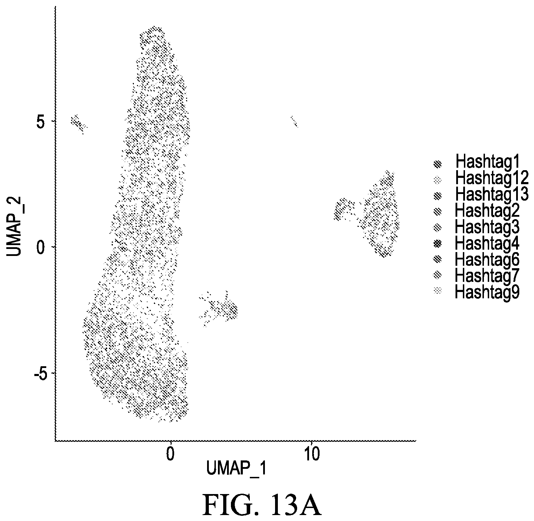

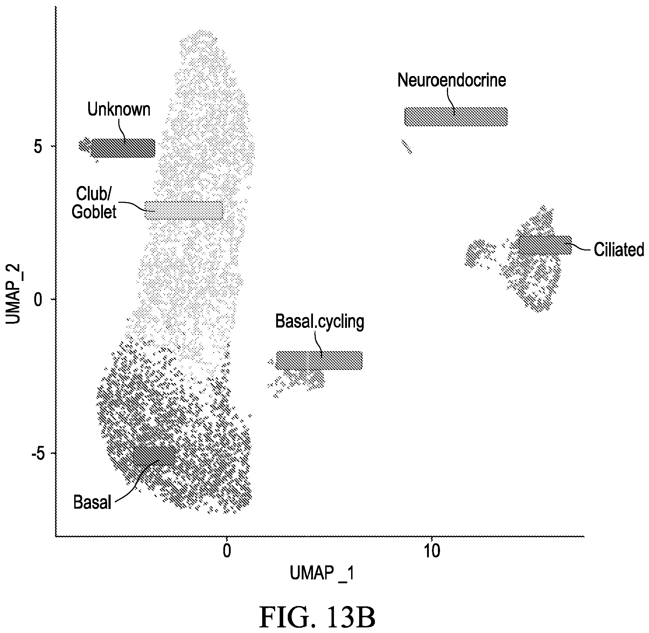

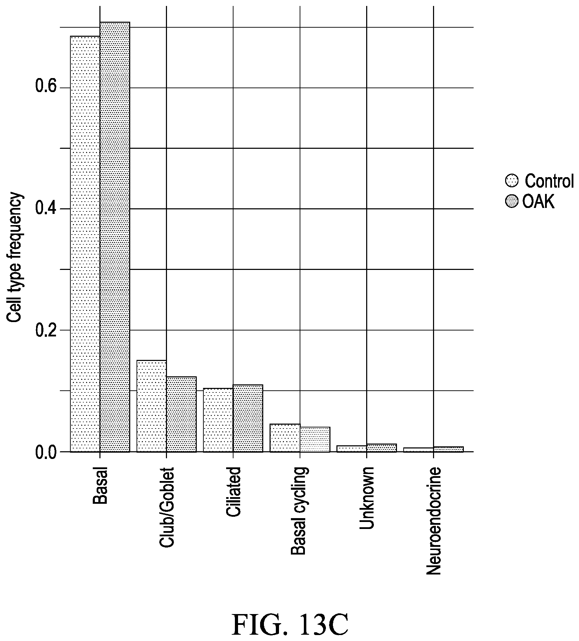

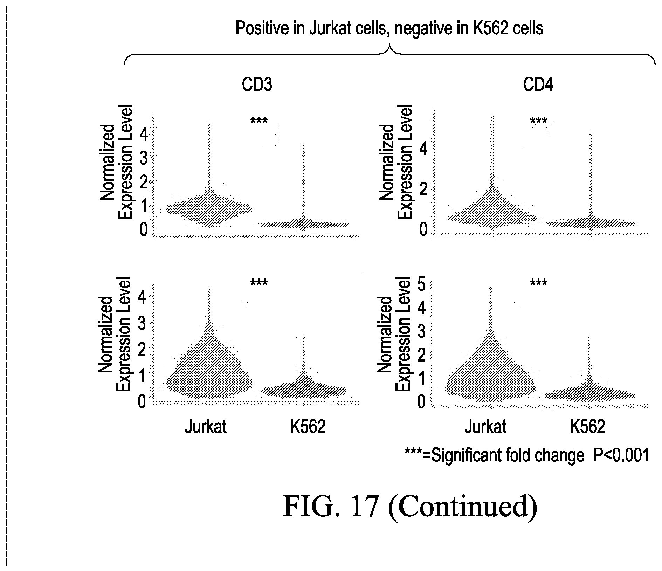

The abundance of cells assigned the 9 different hashtags in the sequencing data obtained using the OAK method and the control method was analyzed. As shown in FIG. 10, a high correlation was observed between the sequencing data obtained from the OAK method and the sequencing data of the control method. These data confirm that the OAK method works well with cell hashing. Ridge plots were then generated to show hashtag expression level for cells deplexed for each hashtag identity. As shown in FIG. 11, the ridge plots demonstrated high signal to noise ratios for each cell assigned using a hashtag. FIG. 12 shows that the quality of the transcriptome sequencing data obtained from the OAK method with cell hashing is comparable to the quality of the transcriptome sequencing data obtained from the control method. UMAP analysis of scRNA-Seq data obtained using the OAK method with hashtags showed that the hashtags were evenly distributed amongst different cell types as expected for non-biased cell indexing (FIGS. 13A-13B) and that the frequency of cell types was comparable between the OAK method and the control method (FIG. 13C).

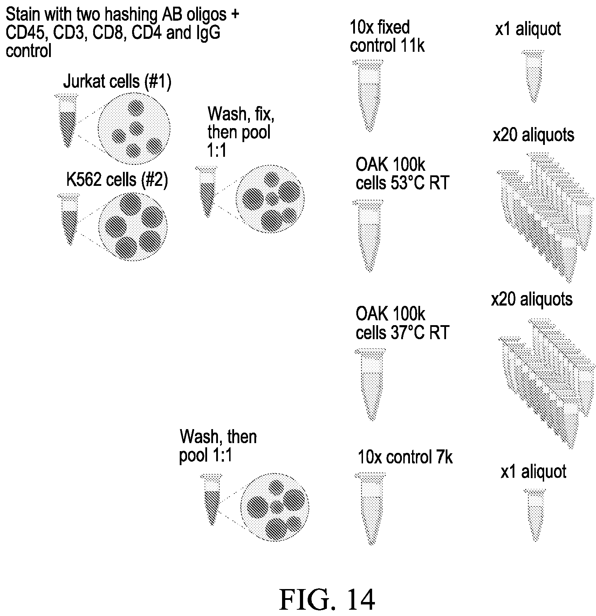

Experiments were further performed to confirm if protein levels can be determined using the OAK methodology with cell hashing. FIG. 14 provides a schematic of the methodology used for this experiment. As shown in FIG. 14, two human cell lines (Jurkat cells and K562 cells) were independently stained with two different hashtag antibodies along with antibodies for proteins known to be differentially expressed between the two cell lines (CD45, CD3 and CD4 conjugated antibodies) and antibodies for proteins known not to be expressed by the two cells lines (RatIgG2b isotype control and CD8 conjugated antibodies). After staining, the cells were combined in a 1:1 ratio, fixed with methanol and then processed using the 10x Chromium single cell 3' reagent kit and the Chromium platform using the OAK method described in Example 1. A secondary index was added with primers to both the antibody hashing library and the gene expression library. Addition of the index to the antibody fraction was either during direct amplification from the cDNA, or after a second PCR to amplify the antibody-derived sequence (as described in Example 3). Two OAK aliquots were sequenced for two different temperature conditions (37°C and 53°C). An unfixed control using the 10x genomics protocol was also carried out on 12,000 cells for comparison.

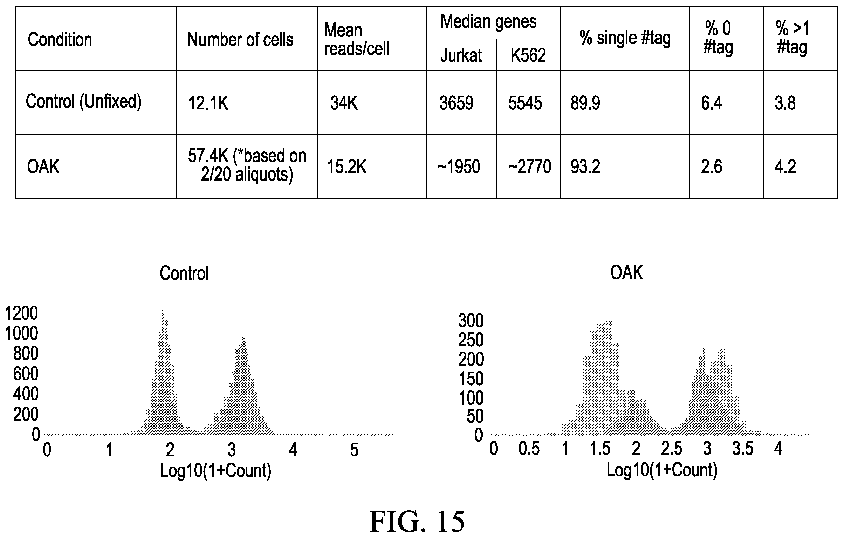

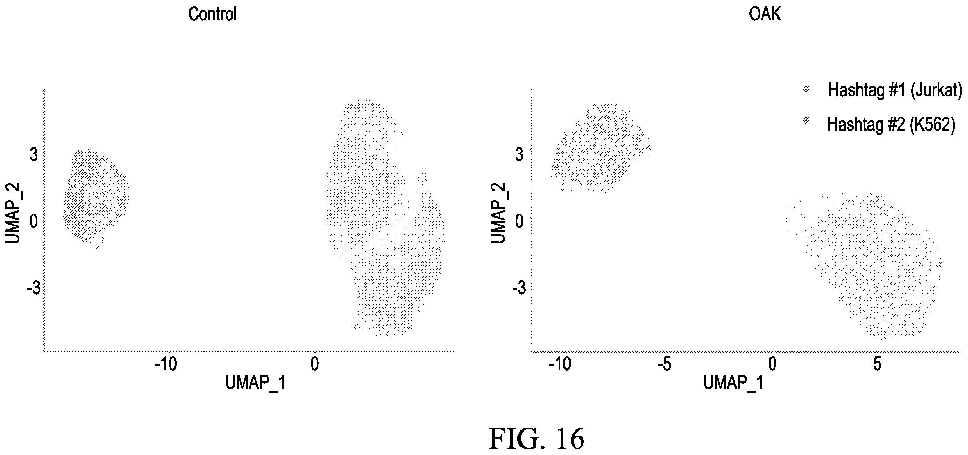

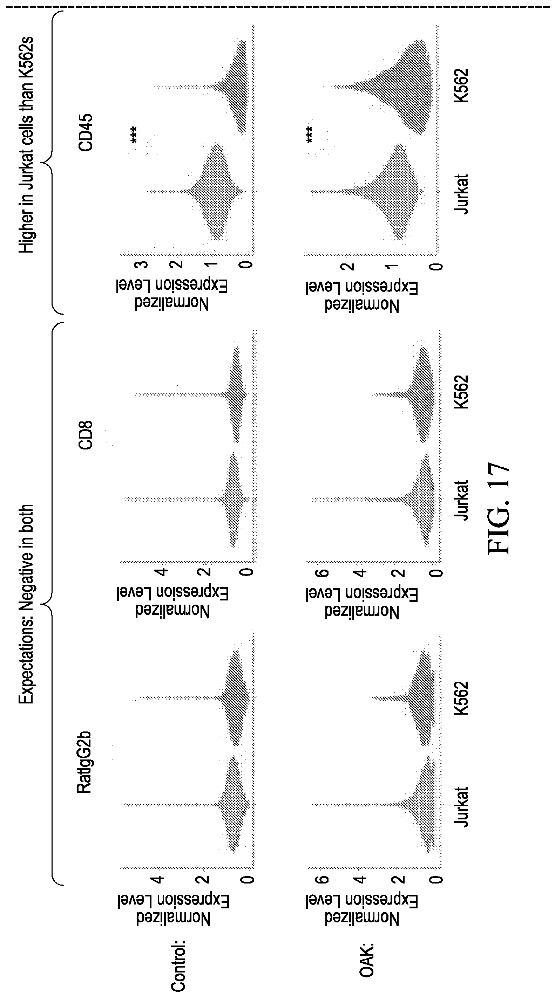

As shown in FIG. 15, the OAK methodology allows for the processing of a large number of cells at a single time. UMAP analysis shown in FIG. 16 illustrates how clustering separates the two cell lines based on scRNA-Seq data and that the clustering matched the assigned hashtags. The expression of the proteins bound by the antibodies was further analyzed and compared with the signal from the antibody hashtags (which identified the cell type). As shown in FIG. 17, signal derived from antibody hashtags matched the expectations for the protein expression in the two cell lines tested.

As shown in this example, cell hashing is compatible with the OAK combinatorial indexing method. In particular, binding of antibodies to specific target proteins was maintained during the fixation process, during exposure to the lysis and reducing agents in the droplets and during the reverse transcription process of the OAK combinatorial indexing method. This example further confirms that cell hashing can be used in combination with the OAK combination indexing and sequencing method to simultaneously analyze protein expression and transcriptomes of single cells. The use of cell hashing in combination with the OAK method can be used to simplify upstream processes for arrays of cells, for increasing the number of cells and for increasing the accuracy of calling multiplets in analysis. Cell hashing can also be used to provide reference abundances for normalizing protein expression data.

### Example 4: OAK Combinatorial Indexing to Detect V(D)J Recombination

This example shows the use of the OAK method for identifying V(D)J recombination patterns in single cells using the combinatorial indexing method.

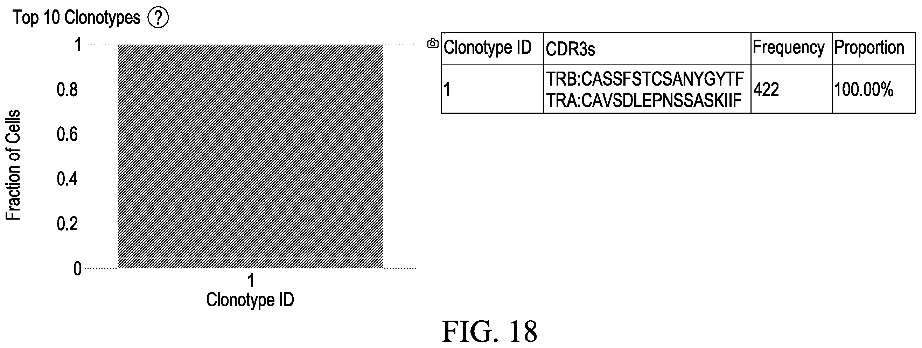

The OAK method described in Example 1 was performed on Jurkat cells with the 10x Chromium single cell 5' reagent kit. cDNA and TCR amplicons were indexed with a second index sequence by aliquot and sequencing libraries were generated. 683 cells of a homogeneous population of Jurkat cells were sequenced and 422 (61.8%) of the cells had the same captured CDR3 sequence as expected, as shown in FIG. 18. The example confirms that the OAK method allows for the sequencing of V(D)J transcripts obtained from single cells.

## Claims

1. A method for combinatorial indexing of nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence; and
(c) incorporating the second index sequence into the nucleic acids in each compartment to generate dual-indexed nucleic acids.
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;
(iv) incorporating the first index sequence into nucleic acids of the cells or nuclei to generate indexed nucleic acids; and
(v) combining the cells or nuclei that associate with or comprise the indexed nucleic acids from a plurality of the emulsion droplets to generate the pooled cells or nuclei associated with or comprising nucleic acids comprising a first index sequence.

2. A method for generating a sequencing library comprising nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence;
(c) incorporating the second index sequence into the nucleic acids in each compartment to generate dual-indexed nucleic acids; and
(d) combining all or a subset of the dual-indexed nucleic acids to generate a sequencing library from the plurality of cells;
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;;
(iv) incorporating the first index sequence into nucleic acids of the cells or nuclei to generate indexed nucleic acids; and
(iv) combining the cells or nuclei that associate with or comprise the indexed nucleic acids from a plurality of the emulsion droplets to generate the pooled cells or nuclei associated with or comprising nucleic acids comprising a first index sequence.

3. A method for sequencing a library comprising nucleic acids from a plurality of single cells, comprising:
(a) providing pooled cells or nuclei associated with or comprising nucleic acids comprising a first index sequence, wherein the first index sequence is incorporated into the nucleic acids of the pooled cells or nuclei in emulsion droplets;
(b) distributing subsets of the pooled cells or nuclei into a plurality of compartments comprising a second index sequence;
(c) incorporating the second index sequence into the nucleic acids in each compartment to generate dual-indexed nucleic acids;
(d) combining all or a subset of the dual-indexed nucleic acids to generate a sequencing library from the plurality of cells; and
(e) sequencing all or a subset of the dual-index nucleic acids;
wherein the first index sequence is incorporated into the nucleic acids of the cells or nuclei in emulsion droplets by a method comprising:
(i) treating the plurality of cells or nuclei with a fixative;
(ii) contacting a the plurality of cells or nuclei with a plurality of particles comprising the first index sequence;
(iii) partitioning individual particles and one or more of the cells or nuclei into emulsion droplets, wherein each emulsion droplet comprises 2 to 20 cells or nuclei;;
(iv) incorporating the first index sequence into nucleic acids of the cells or nuclei to generate indexed nucleic acids; and
(v) combining the cells or nuclei that associate with or comprise the indexed nucleic acids from a plurality of the emulsion droplets to generate the pooled cells or nuclei associated with or comprising nucleic acids comprising a first index sequence.

4. The method of claim 1, wherein the cells or nuclei are treated with a T5 transposase prior to or during step (i).

5. The method of any one of claims 1-4, wherein the plurality of cells are permeabilized or lysed prior to step (i).

6. The method of any one of claims 1-5, wherein the plurality of cells or nuclei are treated with a multiplexing reagent prior to or during step (i).

7. The method of any one of claims 1-6, wherein the first index sequence of each particle or a subset of particles is unique relative to other particles.

8. The method of any one of claims 1-7, wherein the second index sequence in one compartment or a subset of compartments is unique relative to other second index sequences.

9. The method of any one of claims 1-8, wherein the plurality of compartments is a multiwell plate.

10. The method of any one of claims 1-9, wherein the emulsion droplets are generated in an emulsion droplet generating device or generated using an emulsion droplet generating technique.

11. The method of any one of claims 1-10 further comprising incorporating a third index sequence into the dual-indexed nucleic acids to generate triple-indexed nucleic acids.

12. The method of any one of claims 1-11, wherein incorporating the first index sequence into the nucleic acids in emulsion droplets is performed by an amplification process, a reversetranscription process or a ligation process.

13. The method of any one of claims 1-12, wherein incorporating the second index sequence into the indexed nucleic acids is performed by an amplification process or a ligation process.

14. The method of claim 12 or 13, wherein the amplification process is polymerase chain reaction (PCR) or an isothermal amplification process.

15. The method of any one of claims 1-14, wherein the plurality of cells comprises pluripotent stem cells, embryonic stem cells, somatic cells, immune cells, cancer cells or a combination thereof.

## Patentansprüche

1. Verfahren zur kombinatorischen Indexierung von Nukleinsäuren aus einer Vielzahl von Einzelzellen, umfassend:
(a) Bereitstellen von gepoolten Zellen oder Kernen, die mit Nukleinsäuren assoziiert sind oder diese umfassen, umfassend eine erste Indexsequenz, wobei die erste Indexsequenz in die Nukleinsäuren der gepoolten Zellen oder Kerne in Emulsionströpfchen eingebaut wird;
(b) Verteilen von Teilmengen der gepoolten Zellen oder Kerne in eine Vielzahl von Kompartimenten, umfassend eine zweite Indexsequenz; und
(c) Einbauen der zweiten Indexsequenz in die Nukleinsäuren in jedem Kompartiment, um doppelt indexierte Nukleinsäuren zu erzeugen.
wobei die erste Indexsequenz in die Nukleinsäuren der Zellen oder Kerne in Emulsionströpfchen durch ein Verfahren eingebaut wird, umfassend:
(i) Behandeln der Vielzahl von Zellen oder Kernen mit einem Fixiermittel;
(ii) Inkontaktbringen der Vielzahl von Zellen oder Kernen mit einer Vielzahl von Partikeln, umfassend die erste Indexsequenz;
(iii) Aufteilen einzelner Partikel und einer oder mehrerer der Zellen oder Kerne in Emulsionströpfchen, wobei jedes Emulsionströpfchen 2 bis 20 Zellen oder Kerne umfasst;
(iv) Einbauen der ersten Indexsequenz in Nukleinsäuren der Zellen oder Kerne, um indexierte Nukleinsäuren zu erzeugen; und
(v) Kombinieren der Zellen oder Kerne, die mit den indexierten Nukleinsäuren assoziiert sind oder diese umfassen, aus einer Vielzahl der Emulsionströpfchen, um die gepoolten Zellen oder Kerne zu erzeugen, die mit Nukleinsäuren assoziiert sind oder diese umfassen, umfassend eine erste Indexsequenz.

2. Verfahren zum Erzeugen einer Sequenzierungsbibliothek, umfassend Nukleinsäuren aus einer Vielzahl von Einzelzellen, umfassend:
(a) Bereitstellen von gepoolten Zellen oder Kernen, die mit Nukleinsäuren assoziiert sind oder diese umfassen, umfassend eine erste Indexsequenz, wobei die erste Indexsequenz in die Nukleinsäuren der gepoolten Zellen oder Kerne in Emulsionströpfchen eingebaut wird;
(b) Verteilen von Teilmengen der gepoolten Zellen oder Kerne in eine Vielzahl von Kompartimenten, umfassend eine zweite Indexsequenz;
(c) Einbauen der zweiten Indexsequenz in die Nukleinsäuren in jedem Kompartiment, um doppelt indexierte Nukleinsäuren zu erzeugen; und
(d) Kombinieren aller oder einer Teilmenge der doppelt indexierten Nukleinsäuren, um eine Sequenzierungsbibliothek aus der Vielzahl von Zellen zu erzeugen;
wobei die erste Indexsequenz in die Nukleinsäuren der Zellen oder Kerne in Emulsionströpfchen durch ein Verfahren eingebaut wird, umfassend:
(i) Behandeln der Vielzahl von Zellen oder Kernen mit einem Fixiermittel;
(ii) Inkontaktbringen der Vielzahl von Zellen oder Kernen mit einer Vielzahl von Partikeln, umfassend die erste Indexsequenz;
(iii) Aufteilen einzelner Partikel und einer oder mehrerer der Zellen oder Kerne in Emulsionströpfchen, wobei jedes Emulsionströpfchen 2 bis 20 Zellen oder Kerne umfasst;
(iv) Einbauen der ersten Indexsequenz in Nukleinsäuren der Zellen oder Kerne, um indexierte Nukleinsäuren zu erzeugen; und
(iv) Kombinieren der Zellen oder Kerne, die mit den indexierten Nukleinsäuren assoziiert sind oder diese umfassen, aus einer Vielzahl der Emulsionströpfchen, um die gepoolten Zellen oder Kerne zu erzeugen, die mit Nukleinsäuren assoziiert sind oder diese umfassen, umfassend eine erste Indexsequenz.

3. Verfahren zum Sequenzieren einer Bibliothek, umfassend Nukleinsäuren aus einer Vielzahl von Einzelzellen, umfassend:
(a) Bereitstellen von gepoolten Zellen oder Kernen, die mit Nukleinsäuren assoziiert sind oder diese umfassen, umfassend eine erste Indexsequenz, wobei die erste Indexsequenz in die Nukleinsäuren der gepoolten Zellen oder Kerne in Emulsionströpfchen eingebaut wird;
(b) Verteilen von Teilmengen der gepoolten Zellen oder Kerne in eine Vielzahl von Kompartimenten, umfassend eine zweite Indexsequenz;
(c) Einbauen der zweiten Indexsequenz in die Nukleinsäuren in jedem Kompartiment, um doppelt indexierte Nukleinsäuren zu erzeugen;
(d) Kombinieren aller oder einer Teilmenge der doppelt indexierten Nukleinsäuren, um eine Sequenzierungsbibliothek aus der Vielzahl von Zellen zu erzeugen; und
(e) Sequenzieren aller oder einer Teilmenge der doppelt indexierten Nukleinsäuren;
wobei die erste Indexsequenz in die Nukleinsäuren der Zellen oder Kerne in Emulsionströpfchen durch ein Verfahren eingebaut wird, umfassend:
(i) Behandeln der Vielzahl von Zellen oder Kernen mit einem Fixiermittel;
(ii) Inkontaktbringen der Vielzahl von Zellen oder Kernen mit einer Vielzahl von Partikeln, umfassend die erste Indexsequenz;
(iii) Aufteilen einzelner Partikel und einer oder mehrerer der Zellen oder Kerne in Emulsionströpfchen, wobei jedes Emulsionströpfchen 2 bis 20 Zellen oder Kerne umfasst;
(iv) Einbauen der ersten Indexsequenz in Nukleinsäuren der Zellen oder Kerne, um indexierte Nukleinsäuren zu erzeugen; und
(v) Kombinieren der Zellen oder Kerne, die mit den indexierten Nukleinsäuren assoziiert sind oder diese umfassen, aus einer Vielzahl der Emulsionströpfchen, um die gepoolten Zellen oder Kerne zu erzeugen, die mit Nukleinsäuren assoziiert sind oder diese umfassen, umfassend eine erste Indexsequenz.

4. Verfahren nach Anspruch 1, wobei die Zellen oder Kerne vor oder während Schritt (i) mit einer T5-Transposase behandelt werden.

5. Verfahren nach einem der Ansprüche 1-4, wobei die Vielzahl von Zellen vor Schritt (i) permeabilisiert oder lysiert wird.

6. Verfahren nach einem der Ansprüche 1-5, wobei die Vielzahl von Zellen oder Kernen vor oder während Schritt (i) mit einem Multiplexreagens behandelt wird.

7. Verfahren nach einem der Ansprüche 1-6, wobei die erste Indexsequenz jedes Partikels oder einer Teilmenge von Partikeln relativ zu anderen Partikeln einzigartig ist.

8. Verfahren nach einem der Ansprüche 1-7, wobei die zweite Indexsequenz in einem Kompartiment oder einer Teilmenge von Kompartimenten relativ zu anderen zweiten Indexsequenzen einzigartig ist.

9. Verfahren nach einem der Ansprüche 1-8, wobei die Vielzahl von Kompartimenten eine Multiwell-Platte ist.

10. Verfahren nach einem der Ansprüche 1-9, wobei die Emulsionströpfchen in einer Emulsionströpfchen-Erzeugungsvorrichtung erzeugt werden oder unter Verwendung einer Emulsionströpfchen-Erzeugungstechnik erzeugt werden.

11. Verfahren nach einem der Ansprüche 1-10, ferner umfassend das Einbauen einer dritten Indexsequenz in die doppelt indexierten Nukleinsäuren, um dreifach indexierte Nukleinsäuren zu erzeugen.

12. Verfahren nach einem der Ansprüche 1-11, wobei das Einbauen der ersten Indexsequenz in die Nukleinsäuren in Emulsionströpfchen durch einen Amplifikationsprozess, einen Reverse-Transkriptionsprozess oder einen Ligationsprozess durchgeführt wird.

13. Verfahren nach einem der Ansprüche 1-12, wobei das Einbauen der zweiten Indexsequenz in die indexierten Nukleinsäuren durch einen Amplifikationsprozess oder einen Ligationsprozess durchgeführt wird.

14. Verfahren nach Anspruch 12 oder 13, wobei der Amplifikationsprozess eine Polymerasekettenreaktion (PCR) oder ein isothermer Amplifikationsprozess ist.

15. Verfahren nach einem der Ansprüche 1-14, wobei die Vielzahl von Zellen pluripotente Stammzellen, embryonale Stammzellen, somatische Zellen, Immunzellen, Krebszellen oder eine Kombination davon umfasst.

## Revendications

1. Procédé d'indexation combinatoire d'acides nucléiques issus d'une pluralité de cellules uniques, comprenant :
(a) la fourniture de cellules ou noyaux regroupés associés à ou comprenant des acides nucléiques comprenant une première séquence d'index, la première séquence d'index étant incorporée dans les acides nucléiques des cellules ou noyaux regroupés dans des gouttelettes d'émulsion ;
(b) la distribution de sous-ensembles des cellules ou noyaux regroupés dans une pluralité de compartiments comprenant une deuxième séquence d'index ; et
(c) l'incorporation de la deuxième séquence d'index dans les acides nucléiques dans chaque compartiment pour générer des acides nucléiques doublement indexés,
dans lequel la première séquence d'index est incorporée dans les acides nucléiques des cellules ou noyaux dans des gouttelettes d'émulsion par un procédé comprenant :
(i) le traitement de la pluralité de cellules ou noyaux avec un fixateur ;
(ii) la mise en contact de la pluralité de cellules ou noyaux avec une pluralité de particules comprenant la première séquence d'index ;
(iii) la répartition de particules individuelles et d'un(e) ou de plusieurs des cellules ou noyaux dans des gouttelettes d'émulsion, chaque gouttelette d'émulsion comprenant 2 à 20 cellules ou noyaux ;
(iv) l'incorporation de la première séquence d'index dans les acides nucléiques des cellules ou noyaux pour générer des acides nucléiques indexés ; et
(v) la combinaison des cellules ou noyaux qui s'associent aux ou comprennent les acides nucléiques indexés issus d'une pluralité des gouttelettes d'émulsion pour générer les cellules ou noyaux regroupés associés à ou comprenant des acides nucléiques comprenant une première séquence d'index.

2. Procédé de génération d'une bibliothèque de séquençage comprenant des acides nucléiques issus d'une pluralité de cellules uniques, comprenant :
(a) la fourniture de cellules ou noyaux regroupés associés à ou comprenant des acides nucléiques comprenant une première séquence d'index, la première séquence d'index étant incorporée dans les acides nucléiques des cellules ou noyaux regroupés dans des gouttelettes d'émulsion ;
(b) la distribution de sous-ensembles des cellules ou noyaux regroupés dans une pluralité de compartiments comprenant une deuxième séquence d'index ;
(c) l'incorporation de la deuxième séquence d'index dans les acides nucléiques dans chaque compartiment pour générer des acides nucléiques doublement indexés ; et
(d) la combinaison de la totalité ou d'un sous-ensemble des acides nucléiques doublement indexés pour générer une bibliothèque de séquençage à partir de la pluralité de cellules ;
dans lequel la première séquence d'index est incorporée dans les acides nucléiques des cellules ou noyaux dans des gouttelettes d'émulsion par un procédé comprenant :
(i) le traitement de la pluralité de cellules ou noyaux avec un fixateur ;
(ii) la mise en contact de la pluralité de cellules ou noyaux avec une pluralité de particules comprenant la première séquence d'index ;
(iii) la répartition de particules individuelles et d'un(e) ou de plusieurs des cellules ou noyaux dans des gouttelettes d'émulsion, chaque gouttelette d'émulsion comprenant 2 à 20 cellules ou noyaux ;
(iv) l'incorporation de la première séquence d'index dans les acides nucléiques des cellules ou noyaux pour générer des acides nucléiques indexés ; et
(iv) la combinaison des cellules ou noyaux qui s'associent aux ou comprennent les acides nucléiques indexés issus d'une pluralité des gouttelettes d'émulsion pour générer les cellules ou noyaux regroupés associés à ou comprenant des acides nucléiques comprenant une première séquence d'index.

3. Procédé de séquençage d'une bibliothèque comprenant des acides nucléiques issus d'une pluralité de cellules uniques, comprenant :
(a) la fourniture de cellules ou noyaux regroupés associés à ou comprenant des acides nucléiques comprenant une première séquence d'index, la première séquence d'index étant incorporée dans les acides nucléiques des cellules ou noyaux regroupés dans des gouttelettes d'émulsion ;
(b) la distribution de sous-ensembles des cellules ou noyaux regroupés dans une pluralité de compartiments comprenant une deuxième séquence d'index ;
(c) l'incorporation de la deuxième séquence d'index dans les acides nucléiques dans chaque compartiment pour générer des acides nucléiques doublement indexés ;
(d) la combinaison de la totalité ou d'un sous-ensemble des acides nucléiques doublement indexés pour générer une bibliothèque de séquençage à partir de la pluralité de cellules ; et
(e) le séquençage de la totalité ou d'un sous-ensemble des acides nucléiques doublement indexés ;
dans lequel la première séquence d'index est incorporée dans les acides nucléiques des cellules ou noyaux dans des gouttelettes d'émulsion par un procédé comprenant :
(i) le traitement de la pluralité de cellules ou noyaux avec un fixateur ;
(ii) la mise en contact de la pluralité de cellules ou noyaux avec une pluralité de particules comprenant la première séquence d'index ;
(iii) la répartition de particules individuelles et d'un(e) ou de plusieurs des cellules ou noyaux dans des gouttelettes d'émulsion, chaque gouttelette d'émulsion comprenant 2 à 20 cellules ou noyaux ;
(iv) l'incorporation de la première séquence d'index dans les acides nucléiques des cellules ou noyaux pour générer des acides nucléiques indexés ; et
(v) la combinaison des cellules ou noyaux qui s'associent aux ou comprennent les acides nucléiques indexés issus d'une pluralité des gouttelettes d'émulsion pour générer les cellules ou noyaux regroupés associés à ou comprenant des acides nucléiques comprenant une première séquence d'index.

4. Procédé selon la revendication 1, dans lequel les cellules ou noyaux sont traités par une transposase T5 avant ou pendant l'étape (i).

5. Procédé selon l'une quelconque des revendications 1 à 4, dans lequel la pluralité de cellules sont perméabilisées ou lysées avant l'étape (i).

6. Procédé selon l'une quelconque des revendications 1 à 5, dans lequel la pluralité de cellules ou noyaux sont traités avec un réactif de multiplexage avant ou pendant l'étape (i).

7. Procédé selon l'une quelconque des revendications 1 à 6, dans lequel la première séquence d'index de chaque particule ou d'un sous-ensemble de particules est unique par rapport aux autres particules.

8. Procédé selon l'une quelconque des revendications 1 à 7, dans lequel la deuxième séquence d'index dans un compartiment ou un sous-ensemble de compartiments est unique par rapport aux autres deuxièmes séquences d'index.

9. Procédé selon l'une quelconque des revendications 1 à 8, dans lequel la pluralité de compartiments est une plaque multipuits.

10. Procédé selon l'une quelconque des revendications 1 à 9, dans lequel les gouttelettes d'émulsion sont générées dans un dispositif de génération de gouttelettes d'émulsion ou générées en utilisant une technique de génération de gouttelettes d'émulsion.

11. Procédé selon l'une quelconque des revendications 1 à 10 comprenant en outre l'incorporation d'une troisième séquence d'index dans les acides nucléiques doublement indexés pour générer des acides nucléiques triplement indexés.

12. Procédé selon l'une quelconque des revendications 1 à 11, dans lequel l'incorporation de la première séquence d'index dans les acides nucléiques dans des gouttelettes d'émulsion est réalisée par un processus d'amplification, un processus de transcription inverse ou un processus de ligation.

13. Procédé selon l'une quelconque des revendications 1 à 12, dans lequel l'incorporation de la deuxième séquence d'index dans les acides nucléiques indexés est réalisée par un processus d'amplification ou un processus de ligation.

14. Procédé selon la revendication 12 ou 13, dans lequel le processus d'amplification est une réaction en chaîne par polymérase (PCR) ou un processus d'amplification isotherme.

15. Procédé selon l'une quelconque des revendications 1 à 14, dans lequel la pluralité de cellules comprend des cellules souches pluripotentes, des cellules souches embryonnaires, des cellules somatiques, des cellules immunitaires, des cellules cancéreuses ou une combinaison de celles-ci.
